(19) **Europäisches Patentamt / European Patent Office / Office européen des brevets**

(11) **EP 3 550 030 B1**

(12) **EUROPEAN PATENT SPECIFICATION**

(45) Date of publication and mention
of the grant of the patent:
**01.01.2025 Bulletin 2025/01**

(21) Application number: **17875135.0**

(22) Date of filing: **30.11.2017**

(51) International Patent Classification (IPC):
*C12Q 1/28* *(2006.01)*      *C12Q 1/26* *(2006.01)*
*C12Q 1/37* *(2006.01)*      *G01N 33/72* *(2006.01)*

(52) Cooperative Patent Classification (CPC):
**G01N 33/723; C12Q 1/26; C12Q 1/28; C12Q 1/37;**
**G01N 33/72**

(86) International application number:
**PCT/JP2017/042994**

(87) International publication number:
**WO 2018/101389 (07.06.2018 Gazette 2018/23)**

(54) **METHOD FOR MEASURING GLYCATION RATE OF HEMOGLOBIN**

VERFAHREN ZUR MESSUNG DER GLYKIERUNGSRATE VON HÄMOGLOBIN

PROCÉDÉ DE MESURE DU TAUX DE GLYCATION DE L'HÉMOGLOBINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.11.2016   JP 2016232613**
**30.11.2016   JP 2016232614**

(43) Date of publication of application:
**09.10.2019   Bulletin 2019/41**

(73) Proprietor: **TOYOBO CO., LTD.**
**Osaka-shi, Osaka 530-0001 (JP)**

(72) Inventors:
• **MATSUO, Miho**
**Tsuruga-shi**
**Fukui 914-8550 (JP)**

• **MAEHARA, Shiori**
**Tsuruga-shi**
**Fukui 914-8550 (JP)**

(74) Representative: **Vossius & Partner**
**Patentanwälte Rechtsanwälte mbB**
**Siebertstrasse 3**
**81675 München (DE)**

(56) References cited:
**EP-B1- 1 002 874          WO-A1-2008/013874**
**WO-A1-2008/108385          JP-A- 2007 147 630**
**JP-A- 2007 147 630          JP-A- 2009 159 989**
**JP-A- 2009 544 315          JP-A- 2015 165 827**
**US-A1- 2005 221 415**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a method for measuring a glycated hemoglobin ratio. More specifically, the present invention relates to a method for measuring the ratio of glycated hemoglobin in biological samples, such as blood, using enzymatic reactions; and also relates to a composition therefor.

Background Art

Glycated hemoglobin, such as HbA1c, is used as a marker of diabetes. HbAlc is glycated hemoglobin in which glucose binds to the amino group of valine at the β-chain N terminal of hemoglobin. When glycated hemoglobin is used as a diabetes marker, the glycation ratio of hemoglobin, i.e., the ratio of glycated hemoglobin concentration to total hemoglobin concentration (hereafter also referred to as "glycated hemoglobin ratio"), is determined and used for diagnosis. For example, the ratio of HbA1c concentration to total hemoglobin concentration (hereafter also referred to as "HbA1c ratio") is considered to be an excellent diabetes indicator, because the amount of HbAlc does not change rapidly compared with the amount of glucose in blood, and correlates well with blood sugar levels one to two months ago.

As the current method for measuring a glycated hemoglobin ratio, a high-performance chromatography (HPLC) method and latex agglutination immunoturbidimetry have been common. The HPLC method has problems of high column price and low throughput. The latex agglutination immunoturbidimetry has problems such that the reaction vessel of the analysis apparatus used for measurement is contaminated, and such that troublesome maintenance is required. Accordingly, enzymatic processes solving these problems have recently attracted attention and have become common.

A typical enzymatic process used to measure a glycated hemoglobin ratio is such that a total hemoglobin concentration is measured by a non-enzymatic reaction, a glycated hemoglobin concentration is separately measured by an enzymatic reaction, and a glycated hemoglobin ratio is determined from formulas.

Examples of the method for measuring a total hemoglobin concentration include a cyanmethemoglobin method, acid hematin method, alkaline hematin method, oxyhemoglobin method, SLS-hemoglobin method, Hb-red measurement method, and HbCO measurement method, all of which are international standard methods.

The total hemoglobin concentration is determined in such a manner that a total hemoglobin concentration calibration curve is prepared using two calibrators with known different total hemoglobin concentrations, then the total amount of hemoglobin in a sample is measured, and the total hemoglobin concentration is determined using the calibration curve.

In contrast, the method for measuring a glycated hemoglobin concentration is a colorimetry method in which glycated hemoglobin or a decomposition product thereof is allowed to react with an oxidoreductase to produce hydrogen peroxide, the hydrogen peroxide is further allowed to react with a chromogenic substrate in the presence of peroxidase, and the amount of coloring is measured (PTL 1).

The glycated hemoglobin concentration is determined in such a manner that a glycated hemoglobin concentration calibration curve is prepared using two calibrators with known different glycated hemoglobin concentrations, then the amount of glycated hemoglobin in a sample is measured, and the glycated hemoglobin concentration is determined using the calibration curve.

For example, when HbAlc is selected as glycated hemoglobin, the glycated HbAlc ratio can be determined by applying the total hemoglobin concentration and glycated hemoglobin concentration obtained by the above reactions to the following formulas:

HbA1c (NGSP) value = 0.0915 × HbA1c concentration (mmol/L)/total Hb concentration (mol/L) + 2.15

$$\text{HbA1c (JDS) value} = 0.980 \times \text{HbA1c (NGSP) value} - 0.245$$

Accordingly, when the HbAlc ratio measurement method using an enzymatic process is applied to an autoanalyzer, two channels, i.e., a channel for measuring total hemoglobin and a channel for measuring HbA1c, are required; thus, reagents for the two channels are required, which is troublesome. Moreover, because two measurements are carried out, the measurement processing speed of the HbAlc ratio is lower than that of a method of carrying out one measurement using one channel. Moreover, in the prior art, a method for measuring a glycosylated hexapeptide derived from a glycosylated protein has previously been described WO 2008/108385 Al.

Further, a measuring method using a sulfonic acid compound or a nitro compound has been described wherein said sulfonic acid compound or said nitro compound is added to the sample to eliminate influence as reducing substance for hemoglobin and hemoglobin resolvent contained in the sample JP 2007 147630.
Furthermore, a highly reliable method of measuring an analyte in a sample using a redox reaction has recently been described EP 1 002 874.

Citation List

Patent Literature

PTL 1: JP4427137B

Summary of Invention

Technical Problem

The present invention was made against the background of the above problems of the prior art. Specifically, an object of the present invention is to improve a method for measuring a glycated hemoglobin ratio using an enzymatic process to a simpler, faster, and/or more accurate method, and/or to provide a calibrator, a reagent composition, etc., for use in the above measurement method.

Solution to Problem

As a result of extensive research, the present inventors found that the above object can be achieved by the means shown below, and have reached the present invention. Although not wishing to be bound by any theory, it is assumed in the present invention that a strong correlation occurs between a glycated hemoglobin ratio and the amount of coloring obtained by the reaction of hydrogen peroxide and a chromogenic substrate in the presence of peroxidase for the following reason. That is, a compound having a sulfo group is brought into contact with hemoglobin, whereby divalent iron ions contained in the hemoglobin molecules are partially oxidized, so that the reductive capacity is weakened at a predetermined ratio; and the hemoglobin with weakened reductive capacity reduces, depending on the amount thereof, the concentration of hydrogen peroxide produced in the enzymatic reaction of glycated amino acid oxidase etc. with glycated hemoglobin. The present inventors also found that this strong correlation can be further increased by using, as calibrators, glycated hemoglobin calibrators value-assigned by two or more levels of glycated hemoglobin ratios, wherein their total hemoglobin concentrations are at a predetermined ratio. Specifically, the present invention relates to the embodiments as characterized in the claims.
Thus, the invention relates to the following :

1. A method for measuring a glycated hemoglobin ratio, the method comprising the following steps (1) to (5):

(1) bringing a sample containing glycated hemoglobin into contact with a compound having a sulfo group;
(2) allowing glycated amino acid oxidase to act on the sample containing glycated hemoglobin to produce hydrogen peroxide;
(3) bringing the hydrogen peroxide produced in step (2) into contact with a chromogenic substrate in the presence of peroxidase to produce a color-forming dye;
(4) measuring the amount of coloring of the color-forming dye obtained in step (3);

(5-1) creating a calibration curve of a glycated hemoglobin ratio and the amount of coloring using glycated hemoglobin calibrators value-assigned by two or more levels of glycated hemoglobin ratios, wherein the glycated hemoglobin calibrators value-assigned by two or more levels of glycated hemoglobin ratios are such that when the lowest total hemoglobin concentration at the time of completion of value-assignment is regarded as 1, the other total hemoglobin concentrations at the time of completion of value-assignment are 1 to 6 times the lowest total hemoglobin concentration; and
(5-2) applying the amount of coloring obtained in step (4) to the calibration curve created in step (5-1) to determine a glycated hemoglobin ratio.

2. The above measurement method wherein the glycated hemoglobin calibrators value-assigned by two or more levels of glycated hemoglobin ratios used in step (5-1) are such that when the lowest total hemoglobin concentration is regarded as 1, the other total hemoglobin concentrations are 1 to 3 times the lowest total hemoglobin concentration.

3. The above measurement method further comprising the following step (6):

(6) decomposing the glycated hemoglobin into a glycated hemoglobin decomposition product by the action of protease.

4. The above measurement method further comprising the following step (7):
(7) hemolyzing the sample containing glycated hemoglobin.

5. The above measurement method wherein the compound having a sulfo group used in step (1) is at least one compound selected from the group consisting of benzenesulfonic acid, alkyl sulfonic acid, naphthalenesulfonic acid, sulfosuccinic acid, benzothiazoline sulfonic acid, anthraquinone sulfonic acid, camphor sulfonic acid, benzoxadiazole sulfonic acid, derivatives thereof, and salts thereof.

6. The above measurement method wherein the compound having a sulfo group used in step (1) is at least one compound selected from the group consisting of dodecylbenzenesulfonic acid, 3-aminobenzenesulfonic acid, alkyldiphenyl ether sulfonic acid, 4-aminoazobenzene-4'-sulfonic acid, 4-amino-4'-nitrostilbene-2,2'-disulfonic acid, 4,4'-diazostilbene-2,2'-disulfonic acid, sulfanilic acid, 5-sulfoisophthalic acid, 5-amino-2-chlorotoluene-4-sulfonic acid, alkylnaphthalenesulfonic acid, 2-naphthalenesulfonic acid, 8-amino-1-naphthalenesulfonic acid, dodecylnaphthalenesulfonic acid, di-2-ethylhexyl sulfosuccinate, dialkylsulfosuccinic acid, and salts thereof.

7. The above measurement method wherein the chromogenic substrate used in step (3) is a leuco chromogen.

8. The above measurement method wherein the chromogenic substrate used in step (3) is a phenothiazine-based leuco chromogen.

9. The above measurement method wherein the chromogenic substrate used in step (3) is at least one phenothiazine-based leuco chromogen selected from the group consisting of 10-(carboxymethylaminocarbonyl)-3,7-bis(dimethylamino)phenothiazine sodium salt, 10-N-carboxymethylcarbamoyl-3,7-bis(dimethylamino)-10H-phenothiazine, and 10-N-methylcarbamoyl-3,7-bis(dimethylamino)-10H-phenothiazine.

10. The above measurement method wherein the chromogenic substrate used in step (3) is 10-(carboxymethylaminocarbonyl)-3,7-bis(dimethylamino)phenothiazine sodium salt.

11. The above measurement method which is performed in one channel of an autoanalyzer.

12. A kit for measuring a glycated hemoglobin ratio, the kit comprising the following reagents (a) to (e):

(a) a reagent containing a compound having a sulfo group;
(b) a reagent containing glycated amino acid oxidase;
(c) a reagent containing peroxidase;
(d) a reagent containing a chromogenic substrate that produces a color-forming dye upon contact with hydrogen peroxide in the presence of peroxidase; and
(e) glycated hemoglobin calibrators value-assigned by two or more levels of glycated hemoglobin ratios, wherein the glycated hemoglobin calibrators value-assigned by two or more levels of glycated hemoglobin ratios are such that when the lowest total hemoglobin concentration at the time of completion of value-assignment is regarded as 1, the other total hemoglobin concentrations at the time of completion of value-assignment are 1 to 6 times the lowest total hemoglobin concentration.

13. The above measurement kit wherein the glycated hemoglobin calibrators value-assigned by two or more levels of glycated hemoglobin ratios (e) are such that when the lowest total hemoglobin concentration is regarded as 1, the other total hemoglobin concentrations are 1 to 3 times the lowest total hemoglobin concentration.

14. The above measurement kit further comprising the following reagent (f):
(f) a reagent containing protease.

Advantageous Effects of Invention

According to the present invention, it is not necessary to separately measure a total hemoglobin concentration and a glycated hemoglobin concentration; when a glycated hemoglobin ratio is measured by an enzymatic process, only one channel is required in a general-purpose autoanalyzer; and a simpler, faster, and/or more accurate measurement is possible.

Brief Description of Drawings

Fig. 1 shows correlation data between the method of the present invention (when the calibration curve A is applied) and the HPLC method in Example 1.
Fig. 2 shows correlation data between the method of the present invention (when the calibration curve B is applied) and the HPLC method in Example 1.
Fig. 3 shows correlation data between the method of the present invention (when the calibration curve C is applied) and the HPLC method in Example 1.
Fig. 4 shows correlation data between the method of the present invention (when the calibration curve D is applied) and the HPLC method in Example 1.
Fig. 5 shows correlation data between the method of the present invention (when the reagent A shown in Table 2 is used) and the HPLC method in Example 2.
Fig. 6 shows correlation data between the method of the present invention (when the reagent B shown in Table 2 is used) and the HPLC method in Example 2.
Fig. 7 shows correlation data between the method of the present invention (when the reagent C shown in Table 2 is used) and the HPLC method in Example 2.
Fig. 8 shows correlation data between the method of the present invention (when the reagent D shown in Table 2 is used) and the HPLC method in Example 2.
Fig. 9 shows correlation data between the method of the present invention (when the reagent E shown in Table 2 is used) and the HPLC method in Example 2.
Fig. 10 shows correlation data between the method of Comparative Example 1 (when the reagent F shown in Table 2 is used) and the HPLC method.
Fig. 11 shows correlation data between the method of Comparative Example 1 (when the reagent G shown in Table 2 is used) and the HPLC method.
Fig. 12 shows correlation data between the method of the present invention (when the calibration curve E is applied) and the HPLC method in Example 3.
Fig. 13 shows correlation data between the method of the present invention (when the calibration curve F is applied) and the HPLC method in Example 3.
Fig. 14 shows correlation data between the method of the present invention (when the calibration curve G is applied) and the HPLC method in Example 3.
Fig. 15 shows correlation data between the method of the present invention (when the calibration curve H is applied) and the HPLC method in Example 3.

Description of Embodiments

Embodiments of the present invention are described in detail below; however, the present invention is not limited thereto.
The term "to" in the present specification means "or more, or less." For example, "★ to ☆" in the specification means "★ or more and * or less." The term "and/or" in the present specification means one or the other or both.
In the present invention, for enzyme activity that is not described in the specification, the activity of commercial products is basically measured according to measurement methods and units if they are described in the containers, attached manuals or pamphlets, etc. Moreover, for those described in documents, the measurement is performed according to the activity measurement methods described in the documents.
When the definition of activity (measurement method) is unknown, or when procurement of reagents required for the measurement is limited, substrates are suitably selected rationally based on the common knowledge of a person skilled in the art, measurement conditions are determined, and the measurement is then performed.

1. Method for measuring glycated hemoglobin ratio

One embodiment of the present invention is a method for measuring a glycated hemoglobin ratio, the method comprising the following steps (1) to (5):

(1) bringing a sample containing glycated hemoglobin into contact with a compound having a sulfo group;
(2) allowing glycated amino acid oxidase to act on the sample containing glycated hemoglobin to produce hydrogen peroxide;

(3) bringing the hydrogen peroxide produced in step (2) into contact with a chromogenic substrate in the presence of peroxidase to produce a color-forming dye;

(4) measuring the amount of coloring of the color-forming dye obtained in step (3);

(5-1) creating a calibration curve of a glycated hemoglobin ratio and the amount of coloring using glycated hemoglobin calibrators value-assigned by two or more levels of glycated hemoglobin ratios, wherein the glycated hemoglobin calibrators value-assigned by two or more levels of glycated hemoglobin ratios are such that when the lowest total hemoglobin concentration at the time of completion of value-assignment is regarded as 1, the other total hemoglobin concentrations at the time of completion of value-assignment are 1 to 6 times the lowest total hemoglobin concentration; and

(5-2) applying the amount of coloring obtained in step (4) to the calibration curve created in step (5-1) to determine a glycated hemoglobin ratio.

According to the method for measuring a glycated hemoglobin ratio of the present invention, it is not necessary to separately measure a total hemoglobin concentration and a glycated hemoglobin concentration. Accordingly, only one channel is required in a general-purpose autoanalyzer, so that a simpler, faster, and more accurate measurement is possible.

1-1. Measurement target, sample, etc.

The sample serving as the measurement target of the method for measuring a glycated hemoglobin ratio of the present invention is not limited. Examples of applicable samples include whole blood, plasma, serum, and blood cells; biological samples (i.e., samples obtained from organisms), such as urine and cerebrospinal fluid; drinking water, such as juice; and foods, such as soy sauce and sauce.

The glycated hemoglobin targeted by the measurement method of the present invention is not limited. For example, glycated hemoglobin in which a sugar binds to the β-chain of hemoglobin is called hemoglobin Al, which is further fractionated into A1a1, A1a2, A1b, and Ale, depending on the type of sugar. In general, one in which fructose 1-6 diphosphate binds to the β-chain of hemoglobin is called hemoglobin A1a1 (HbA1a1), one in which glucose-6-phosphate binds to the β-chain of hemoglobin is called hemoglobin A1a2 (HbA1a2), one in which pyruvic acid binds to the β-chain of hemoglobin is called hemoglobin Alb (HbA1b), and one in which glucose binds to the β-chain of hemoglobin is called hemoglobin Alc (HbA1c). The A1c fraction (HbA1c) is the most common among these fractions, and is often used as a diabetes marker. Although any of the above fractions may be used as the measurement target in the present invention, HbAlc is preferable.

The method of the present invention can be applied to diabetes diagnosis, and is thus useful particularly for whole blood samples and hemocyte preparations among the above samples. Although it is not limited, when glycated hemoglobin in erythrocytes is measured, whole blood may be hemolyzed as it is, or erythrocytes isolated from whole blood may be hemolyzed, and the resulting hemolysis samples may be used as measurement samples.

When a measurement sample is prepared, dilution operation may be performed. The diluent may be purified water or a buffer, such as Good's buffer or phosphate buffer. Amino acids, acid compounds, and sugars, such as saponin and cyclodextrin, may also be contained as stabilizing agents. In addition, it is also possible to use a liquid containing certain components, such as preservatives (e.g., sodium azide and ProClin), chelating agents (e.g., EDTA), and surfactants (e.g., polyoxyethylene alkyl ethers, lauryl trimethylammonium salts, and alkyl betaine).

In a broad sense, samples also include calibrators.

Examples of calibrators include, but are not limited to, whole blood, blood cells, lysates obtained by lysing whole blood in purified water or reagents, lysates obtained by lysing blood cells in purified water or reagents, and the like. The reagents for lysis may be purified water or diluents exemplified above for the purpose of preparing measurement samples. These are preferably refrigerated or frozen-stored, and more preferably frozen-stored. A calibrator frozen by frozen storage may be thawed when used.

Other usable calibrators are those obtained by freeze-drying the above-mentioned whole blood, blood cells, lysates, etc. A freeze-dried calibrator may be redissolved when used. For the redissolution, for example, purified water or diluents exemplified above for the purpose of preparing measurement samples can be used.

1-2. Step (7): Hemolysis etc.

The method for measuring a glycated hemoglobin ratio of the present invention may further comprise the following step (7) :

(7) hemolyzing the sample containing glycated hemoglobin.

Step (7) is preferably used when the sample containing glycated hemoglobin contains whole blood or blood cells.

Step (7) can be performed, for example, during preparation of the measurement sample, prior to steps (1) to (5), as mentioned above. Moreover, step (7) may be performed simultaneously with step (1) prior to steps (2) to (5). Alternatively, after step (7) is performed, step (1) may be performed prior to steps (2) to (5).

When application to an autoanalyzer is taken into consideration, step (7) can be performed during reagent addition, rather than during preparation of the measurement sample. In this case, step (7) is preferably performed in the first step that is started by the first reagent addition.

Hemolysis refers to a phenomenon in which the erythrocyte membrane is broken. Normal whole blood, and erythrocytes floated in an isotonic solution, such as physiological saline, are not hemolyzed and are observed as a red opaque suspension; however, when hemolysis occurs, hemoglobin leaks, and the suspension changes to a red transparent solution.

The hemolysis method is not limited, and examples include a method of suspending whole blood or blood cells in distilled water or a hypotonic solution, so that the erythrocyte membrane is broken using osmotic pressure difference; a method of suspending whole blood or blood cells in a reagent containing a surfactant, so that the erythrocyte membrane is lysed; a method using ultrasonic waves; a method using freezing dissolution; and the like. Of these, a method using a surfactant, or a method using osmotic pressure difference is preferable because of their simple operation etc.

The surfactant used in the above hemolysis method using a surfactant is not limited. Usable examples include nonionic surfactants, such as polyoxyethylene-p-t-octylphenyl ether (e.g., Triton-type surfactants), polyoxyethylene sorbitan alkyl ester (e.g., Tween-type surfactants), and polyoxyethylene alkyl ether (e.g., Brij-type surfactants). Specific examples include TritonX-100, Tween-20, and Brij35 (all of which are trade names), and the like. Moreover, it is also possible to use amphoteric ionic surfactants, such as CHAPSO and CHAPS; anionic surfactants, such as sodium cholate and sodium deoxycholate; and cationic surfactants, such as benzethonium chloride. When the blood cell concentration of the treatment solution is 0.5 to 10 volume%, the treatment conditions using a surfactant mentioned above are generally such that the surfactant is added so that the concentration thereof in the treatment solution is 0.05 to 5 wt.%, and the mixture is stirred at room temperature for about 5 seconds to 1 minute.

When the osmotic pressure difference is used, for example, purified water in an amount 2 to 100 times the volume of whole blood is added to induce hemolysis.

1-3. Step (1): Compound having a sulfo group, etc.

In step (1), a compound having a sulfo group is brought into contact with a sample containing glycated hemoglobin.

Examples of the compound having a sulfo group include, but are not limited to, alkyl sulfonic acid, and salts or derivatives thereof; benzenesulfonic acid, and salts or derivatives thereof; naphthalenesulfonic acid, and salts or derivatives thereof; sulfosuccinic acid, and salts or derivatives thereof; benzothiazoline sulfonic acid, and salts or derivatives thereof; anthraquinone sulfonic acid, and salts or derivatives thereof; camphor sulfonic acid, and salts or derivatives thereof; benzoxadiazole sulfonic acid, and salts or derivatives thereof; and the like. In the present invention, from the viewpoint that the glycated hemoglobin ratio can be obtained much more effectively, benzenesulfonic acid, naphthalenesulfonic acid, sulfosuccinic acid, and derivatives and salts thereof are preferably used; and benzenesulfonic acid, and derivatives and salts thereof are more preferably used. The type of salt is not limited. Examples include sodium salts, potassium salts, and the like; and preferably sodium salts.

Specific examples of alkyl sulfonic acid, and salts or derivatives thereof include, but are not limited to, sodium lauryl sulfate, lithium lauryl sulfate, and the like.

Specific examples of benzenesulfonic acid, and salts or derivatives thereof include, but are not limited to, sodium dodecylbenzenesulfonate, 3-aminobenzenesulfonic acid, sodium 4-aminoazobenzene-4'-sulfonate, disodium 4-amino-4'-nitrostilbene-2,2'-disulfonate, disodium 4,4'-diazostilbene-2,2'-disulfonate, sulfanilic acid, monosodium 5-sulfoisophthalate, 5-amino-2-chlorotoluene-4-sulfonic acid, sodium alkyl diphenyl ether sulfonate, and the like. Preferable examples of benzenesulfonic acid, and derivatives or salts thereof include dodecylbenzenesulfonic acid, 3-aminobenzenesulfonic acid, alkyl diphenyl ether sulfonic acid, and derivatives and salts thereof.

Specific examples of naphthalenesulfonic acid, and salts or derivatives thereof include, but are not limited to, sodium alkylnaphthalenesulfonate, sodium 2-naphthalenesulfonate, 8-amino-1-naphthalenesulfonic acid, sodium dodecyl naphthalenesulfonate, and the like. Alkyl naphthalenesulfonic acid, and derivatives and salts thereof are preferable.

Specific preferable examples of sulfosuccinic acid, and salts or derivatives thereof include, but are not limited to, sodium di-2-ethylhexyl sulfosuccinate, sodium dialkylsulfosuccinate, and the like.

Specific examples of camphor sulfonic acid, and salts or derivatives thereof include, but are not limited to, ($\pm$)-10-camphor sulfonic acid.

Specific examples of benzoxadiazole sulfonic acid, and salts or derivatives thereof include, but are not limited to, 4-chloro-7-chlorosulfonyl-2,1,3-benzoxadiazole.

Particularly preferable among these are sodium lauryl sulfate, sodium dodecylbenzenesulfonate, sodium di-2-ethylhexyl sulfosuccinate, and sodium alkyl diphenyl ether sulfonate; and more preferable is sodium alkyl diphenyl ether sulfonate.

The concentration of the above compound added is not limited. When the blood cell concentration of the treatment solution is 0.01 to 10 volume%, the above compound is preferably added so that the concentration thereof in the treatment solution is 0.05 to 5 wt.%.

For example, when step (1) is performed simultaneously with step (7), a reagent containing 0.25 to 1 volume% of the above compound can be used. Moreover, for example, when step (1) is performed simultaneously with step (2), a reagent containing 0.1 to 3 volume% of the above compound can be used.

In step (1), the method of bringing the above compound into contact with the sample containing glycated hemoglobin is not limited. For example, when a sample containing glycated hemoglobin is prepared, additives may be added to the sample.

In this case, step (1) may be performed simultaneously with or after step (7). Moreover, steps (1) and (2) can be performed simultaneously.

When application to an autoanalyzer is taken into consideration, the reagent components required for step (1) may be all prepared simultaneously with the reagent addition step in which all the reagent components required for step (3) are prepared, or in a previous reagent addition step. For example, for application to an autoanalyzer that can set two steps of reagent addition, the reagent components required for step (1) may be supplied in the first step that is started by the first reagent addition.

1-4. Step (2): Glycated amino acid oxidase etc.

In step (2), glycated amino acid oxidase is allowed to act on the sample containing glycated hemoglobin to produce hydrogen peroxide.

In the present specification, glycated amino acid oxidase refers to an enzyme that can catalyze the reaction to produce hydrogen peroxide by oxidation of an Amadori compound. Glycated amino acid oxidase is also referred to as fructosyl amino acid oxidase, fructosyl peptide oxidase, fructosyl amine oxidase, amadoriase, ketoamine oxidase, glycated hexapeptide oxidase, glycated hemoglobin oxidase, etc.

Examples of the Amadori compound include glycated hemoglobin, which is a glycated protein; glycated dipeptide, which is a glycated hemoglobin decomposition product (e.g., α-fructosyl valyl histidine is known), glycated hexapeptide, which is a glycated hemoglobin decomposition product (e.g., α-fructosyl valyl histidyl leucyl threonyl prolyl glutamic acid is known); glycated amino acid, which is a glycated hemoglobin decomposition product; and the like.

The glycated amino acid oxidase used in the method of the present invention is not limited. Usable examples include known enzymes derived from the genera Achaetomiella, Achaetomium, Thielavia, Chaetomium, Gelasinospora, Microascus, Coniochaeta, Eupenicillium, Pyrenochaeta, Arthrinium, Curvularia, Neocosmospora, Cryptococcus, Phaeosphaeria, Aspergillus, Emericella, Ulocladium, Penicillium, Fusarium, Leptosphaeria, Ophiobolus, Pleospora, Coniochaetidium, Pichia, Debaryomyces, Corynebacterium, Agrobacterium, Arthrobacter, and Phaeosphaeria; and variants thereof.

The glycated amino acid oxidase to react with glycated hexapeptide is not limited. Examples include one disclosed in WO08/108385 as glycosylated hexapeptide oxidase.

Moreover, the glycated amino acid oxidase to react with glycated protein is not limited. Examples include one disclosed in WO2015/005257 and WO2015/005258 as glycated hemoglobin oxidase, and one disclosed in WO2015/060429 as amadoriase.

Usable examples of commercial products include FPO-301 (trademark) (Toyobo Co., Ltd.), FPOX-CE (trademark) (Kikkoman Corporation), FPOX-EE (trademark) (Kikkoman Corporation), and the like.

Glycated amino acid oxidase that cannot be used in the present invention for now will be usable in the present invention if it is modified by genetic engineering or chemical methods in the future so that it is improved to have the above characteristics. Alternatively, glycated amino acid oxidase that will be newly obtained in the future and that has the above characteristics can be used in the present invention. The technical idea of the present invention also includes a measurement system of glycated hemoglobin ratio constructed using such glycated amino acid oxidase.

The conditions that allow glycated amino acid oxidase to act may be any conditions, as long as the glycated amino acid oxidase to be used can act on the measurement target, i.e., glycated peptide, glycated hemoglobin, or a decomposition product thereof, to produce hydrogen peroxide.

The amount of glycated amino acid oxidase used is suitably selected depending on the amount of the measurement target contained in the sample, treatment conditions, or the like. For example, glycated amino acid oxidase is added so that the concentration thereof in the reaction mixture at the time of completion of measurement is 0.1 KU/L or more (preferably 1 KU/L or more, more preferably 2 KU/L or more, and even more preferably 3 KU/L or more), and so that the concentration thereof in the reaction mixture at the time of completion of measurement is 100 KU/L or less (preferably 50 KU/L or less, more preferably 20 KU/L or less, even more preferably 10 KU/L or less, and still more preferably 5 KU/L or less) .

The pH when the glycated amino acid oxidase is allowed to act may not be adjusted, or may be adjusted to a pH suitable for the action of enzyme using, for example, a suitable pH adjuster (e.g., hydrochloric acid, acetic acid, sulfuric acid, sodium hydroxide, or potassium hydroxide) so that the pH of the reaction mixture is 2 or more (preferably 3 or more, more preferably 4 or more, and even more preferably 5 or more), and so that the pH of the reaction mixture is 9 or less (preferably 8.5 or less, and more preferably 8 or less) .

The temperature in which the glycated amino acid oxidase is allowed to act is, for example, 20°C or more (preferably 30°C or more, and more preferably 35°C or more) in the reaction mixture, and 70°C or less (preferably 60°C or less, more preferably 50°C or less, and even more preferably 40°C or less) in the reaction mixture.

The reaction time in this case may be the time sufficient to act on the measurement target, i.e., glycated peptide, glycated hemoglobin, or a decomposition product thereof, to produce hydrogen peroxide. For example, the reaction time is 5 seconds or more (preferably 1 minute or more, and more preferably 3 minutes or more), and 180 minutes or less (preferably 60 minutes or less, and more preferably 10 minutes or less) .

Moreover, when the glycated amino acid oxidase is allowed to act on the sample containing glycated hemoglobin, a modifying agent, such as a surfactant, may be allowed to coexist for the purpose of, for example, enhancing activity.

In step (2), the method for allowing glycated amino acid oxidase to act on the sample containing glycated hemoglobin is not limited. For example, glycated amino acid oxidase may be added to the sample containing glycated hemoglobin.

In this case, as described in the explanation of step (1), steps (1) and (2) can be performed simultaneously. Accordingly, step (2) may be performed simultaneously with or after step (7).

When application to an autoanalyzer is taken into consideration, the reagent components required for step (2) may be all prepared simultaneously with the reagent addition step in which all the reagent components required for step (3) are prepared, or in a previous reagent addition step. For example, for application to an autoanalyzer that can set two steps of reagent addition, the reagent components required for step (2) may be supplied so that all of them are prepared in the first step that is started by the first reagent addition (for example, this embodiment is preferable when an "elimination system," described later, is constructed) ; however, they may be supplied so that they are all prepared in the second step. When the reagent components required for step (2) are supplied so that they are all prepared in the second step, they may be supplied separately in the first and second steps.

For example, for application to an autoanalyzer that can set only one step of reagent addition, the reagent components required for step (2) and the reagent components required for step (3) may be supplied in the same step.

For application to an autoanalyzer that can set three or more steps of reagent addition, the method for supplying reagents may be suitably determined in consideration of the above principle.

1-5. Step (6): Protease etc.

When the reactivity of the glycated amino acid oxidase used in step (2) to glycated protein is low or almost none, the method of the present invention may further comprise the following step (6):

(6) decomposing the glycated hemoglobin into a glycated hemoglobin decomposition product by the action of protease.

In the present specification, protease refers to an enzyme that can act on glycated hemoglobin to catalyze the reaction to decompose it into a glycated hemoglobin decomposition product. Protease is also called peptidase, proteinase, or the like.

The above glycated hemoglobin decomposition product is not limited, as long as it is a product obtained by fragmenting glycated hemoglobin, by protease, into glycated amino acid and/or glycated peptide. The size of the fragments is not limited either. Examples include glycated dipeptide (e.g., $\alpha$-fructosyl valyl histidine), glycated hexapeptide (e.g., $\alpha$-fructosyl valyl histidyl leucyl threonyl prolyl glutamic acid), glycated amino acid, and the like.

The protease used in the method of the present invention is not limited. Examples include amino peptidase, dipeptidase, dipeptidyl peptidase, tripeptidyl peptidase, peptidyl dipeptidase, serine carboxypeptidase, proline carboxypeptidase, alanine carboxypeptidase, metal protease, cysteine carboxypeptidase, omega peptidase, serine end peptidase, cysteine protease, aspartic protease, neutral protease, threonine end peptidase, and the like.

For these proteases, commercially available products may be used as they are, or those produced according to known documents may be used. These proteases may be used in combination of two or more.

In terms of stability, reaction rate (specific activity), easy availability, etc., the protease used in the present invention is preferably the following.

Usable examples include metalloprotease, serine protease, serine carboxypeptidase, proteinase K, bromelain, papain, pig pancreas-derived trypsin, Bacillus subtilis-derived protease, Aspergillus oryzae-derived protease, Aspergillus saitoi-derived protease, Streptomyces griseus-derived protease, and the like.

Examples of commercial products include Protease A "Amano" G (Amano Enzyme Inc.), Protease M "Amano" G (Amano Enzyme Inc.), Protease S "Amano" G (Amano Enzyme Inc.), Protease N "Amano" (Amano Enzyme Inc.), Peptidase R (Amano Enzyme Inc.), Papain M-40 (Amano Enzyme Inc.), Thermolysin (Daiwa Fine Chemicals Co,. Ltd.), Thermoase PC10 (Daiwa Fine Chemicals Co., Ltd.), Toyozyme (Toyobo), pronase (Roche), protease derived from Bacillus sp.(Sigma-Aldrich), protease derived from Streptomyces griseus Type XIV (Sigma-Aldrich), protease derived from Streptomyces griseus Type XXI (Sigma-Aldrich), Actinase E (Kaken Pharmaceutical Co., Ltd.), and Endoproteinase Glu-C (Roche).

Protease that cannot be used in the present invention for now will be usable in the present invention if it is modified by genetic engineering or chemical methods in the future so that it is improved to have the above characteristics. Alternatively, protease that will be newly obtained in the future and that has the above characteristics can be used in the present invention. The technical idea of the present invention also includes a measurement system of glycated hemoglobin ratio constructed using such protease.

The conditions that allow protease to act on glycated hemoglobin may be any conditions, as long as the protease to be used can act on glycated hemoglobin, which is the measurement target, to decompose it into a glycated hemoglobin decomposition product.

The amount of protease used is suitably selected depending on the amount of the measurement target contained in the sample, treatment conditions, or the like. For example, protease is added so that the concentration thereof in the reaction mixture at the time of completion of measurement is 0.01 KU/L or more, generally 0.1 KU/L or more, and more generally 50 KU/L or more (preferably 100 KU/L or more, more preferably 300 KU/L or more, and even more preferably 500 KU/L or more), and so that the concentration thereof in the reaction mixture at the time of completion of measurement is 2000 KU/L or less (preferably 1800 KU/L or less, more preferably 1500 KU/L or less, and even more preferably 1300 KU/L or less) .

The pH when protease is allowed to act may not be adjusted, or may be adjusted to a pH suitable for the action of protease using, for example, a suitable pH adjuster (e.g., hydrochloric acid, acetic acid, sulfuric acid, sodium hydroxide, or potassium hydroxide) so that the pH of the reaction mixture is 2 or more (preferably 3 or more, more preferably 4 or more, and even more preferably 5 or more), and so that the pH of the reaction mixture is 9 or less (preferably 8.5 or less, and more preferably 8 or less).

The temperature in which the protease is allowed to act is, for example, 20°C or higher (preferably 30°C or higher, and more preferably 35°C or higher) in the reaction mixture, and 70°C or lower (preferably 60°C or lower, more preferably 50°C or lower, and even more preferably 40°C or lower) in the reaction mixture.

The treatment time in this case may be the time sufficient to act on glycated hemoglobin, which is the measurement target, to decompose it into a glycated hemoglobin decomposition product. For example, the treatment time is 5 seconds or more (preferably 1 minute or more, and more preferably 3 minutes or more), and is 180 minutes or less (preferably 60 minutes or less, and more preferably 10 minutes or less).

The method for allowing protease to act on glycated hemoglobin in step (6) is not limited. For example, protease may be added to the sample containing glycated hemoglobin.

When application to an autoanalyzer is taken into consideration, the way of thinking for the approach to set the implementation procedure of step (6) differs between when a measurement system in which, in order to eliminate the influence of glycated amino acid, which coexists with the measurement target and is not a measurement target, glycated amino acid oxidase is previously added to eliminate the glycated amino acid, which is not a measurement target (elimination reaction), and the main reaction of glycated hemoglobin, which is the measurement target, is started (hereinafter referred to as the "elimination system") is constructed, and when the elimination system is not constructed.

When the elimination system is not constructed, the reagent components required for step (6) may be all prepared simultaneously with the reagent addition step in which all the reagent components required for step (2) are prepared, or in a previous reagent addition step. For example, for application to an autoanalyzer that can set two steps of reagent addition, the reagent components required for step (6) may be supplied so that they are all prepared in the first step that is started by the first reagent addition; however, they may be supplied so that they are all prepared in the second step. When the reagent components required for step (6) are supplied so that they are all prepared in the second step, they may be separately supplied in the first step and the second step.

For example, for application to an autoanalyzer that can set only one step of reagent addition, the reagent components required for step (6) and the reagent components required for step (2) may be supplied in the same step.

In contrast, when the elimination system is constructed, for application to an autoanalyzer that can set two steps of reagent addition, the elimination reaction is performed in the first step, and the main reaction is performed in the second step; thus, the reagent components required for step (6) are all prepared in the second step, and both the reagent components required for step (6) and the reagent components required for step (2) may be all prepared at this time. In this case, the reagent components required for step (6) may be separately supplied in the first step and the second step, and the reagent components required for step (2) may be separately supplied in the first step and the second step.

For application to an autoanalyzer that can set three or more steps of reagent addition, the method for supplying reagents may be suitably determined in consideration of the above principle.

1-6. Step (3): Peroxidase, chromogenic substrate, etc.

In step (3), the hydrogen peroxide produced in step (2) is brought into contact with a chromogenic substrate in the presence of peroxidase to produce a color-forming dye.

The peroxidase used in the method of the present invention is not limited, as long as the hydrogen peroxide can produce a color-forming dye upon contact with a chromogenic substrate. Preferable examples include those derived from horseradish, microorganisms, etc. Among them, peroxidase derived from horseradish is preferable. The above peroxidase is commercially available with high purity at low cost.

The chromogenic substrate is not limited, as long as it is a chromogen that reacts with hydrogen peroxide in the presence of peroxidase to singly produce a dye. Examples include leuco chromogens, triphenylmethane derivatives, phenothiazine derivatives, diphenylamine derivatives, and the like. Leuco chromogens are preferably used, and phenothiazine-based leuco chromogens are more preferably used.

Specific examples include N-(carboxymethylaminocarbonyl)-4,4'-bis(dimethylamino)diphenylamine sodium salt (abbreviated as DA-64), 10-(carboxymethylaminocarbonyl)-3,7-bis(dimethylamino)phenothiazine sodium salt (abbreviated as DA-67), 10-N-carboxymethylcarbamoyl-3,7-bis(dimethylamino)-10H-phenothiazine (abbreviated as CCAP), 10-N-methylcarbamoyl-3,7-bis(dimethylamino)-10H-phenothiazine (abbreviated as MCDP), 4,4'-bis(dimethylamino)diphenylamine, bis [3-bis (4-chlorophenyl)methyl-4-dimethylaminophenyl] amine (abbreviated as BCMA), N,N,N',N',N'',N''-hexa-3-sulfopropyl-4,4',4''-triaminotriphenylmethane (abbreviated as TPM-PS), 4,4'-benzylidene-bis(N,N-dimethylaniline), and the like.

DA-64 is preferable in terms of less susceptibility to the influence of co-existing substances derived from samples, and DA-67 is preferable in terms of sensitivity. In the present invention, from the viewpoint that the glycated hemoglobin ratio can be obtained much more effectively, it is preferable to use phenothiazine-based leuco chromogens, such as DA-67 (10-(carboxymethylaminocarbonyl)-3,7-bis(dimethylamino)phenothiazine sodium salt), CCAP (10-N-carboxymethylcarbamoyl-3,7-bis(dimethylamino)-10H-phenothiazine), and MCDP (10-N-methylcarbamoyl-3,7-bis(dimethylamino)-10H-phenothiazine); and it is more preferable to use DA-67 (10-(carboxymethylaminocarbonyl)-3,7-bis(dimethylamino)phenothiazine sodium salt).

The amount of chromogenic substrate used is suitably selected depending on the amount of the measurement target contained in the sample, treatment conditions, or the like. For example, 10-(carboxymethylaminocarbonyl)-3,7-bis(dimethylamino)phenothiazine sodium salt is added so that the concentration thereof in the reaction mixture at the time of completion of measurement is 0.001 mmol/L or more (preferably 0.003 mmol/L or more, and more preferably 0.005 mmol/L or more), and so that the concentration thereof in the reaction mixture at the time of completion of measurement is 1.0 mmol/L or less (preferably 1 mmol/L or less, more preferably 0.1 mmol/L or less, and even more preferably 0.05 mmol/L or less). When other chromogenic substrates are used, particularly when phenothiazine-based leuco chromogens are used as chromogenic substrates, they can be used at the same concentration as above.

The conditions that allow peroxidase to act in step (3) may be any conditions, as long as the peroxidase used can act on hydrogen peroxide and a chromogenic substrate to produce a color-forming dye.

The amount of peroxidase used is suitably selected depending on the amount of the measurement target contained in the sample, treatment conditions, or the like. For example, peroxidase is added so that the concentration thereof in the reaction mixture at the time of completion of measurement is 0.1 KU/L or more (preferably 1 KU/L or more, more preferably 2 KU/L or more, and even more preferably 3 KU/L or more), and so that the concentration thereof in the reaction mixture at the time of completion of measurement is 100 KU/L or less (preferably 50 KU/L or less, more preferably 30 KU/L or less, even more preferably 20 KU/L or less, and still more preferably 10 KU/L or less).

The pH when the peroxidase is allowed to act may not be adjusted, or may be adjusted to a pH suitable for the action of peroxidase using, for example, a suitable pH adjuster (e.g., hydrochloric acid, acetic acid, sulfuric acid, sodium hydroxide, or potassium hydroxide) so that the pH of the reaction mixture is 2 or more (preferably 3 or more, more preferably 4 or more, and even more preferably 5 or more), and so that the pH of the reaction mixture is 9 or less (preferably 8.5 or less, and more preferably 8 or less).

The temperature in which the peroxidase is allowed to act is, for example, 20°C or higher (preferably 30°C or higher, and more preferably 35°C or higher) in the reaction mixture, and 70°C or lower (preferably 60°C or lower, more preferably 50°C or lower, and even more preferably 40°C or lower) in the reaction mixture.

The treatment time in this case may be the time sufficient for peroxidase to act on hydrogen peroxide and a chromogenic substrate to produce a color-forming dye. For example, the treatment time is 5 seconds or more (preferably 1 minute or more, and more preferably 3 minutes or more), and is 180 minutes or less (preferably 60 minutes or less, and more preferably 10 minutes or less) .

In step (3), the method for allowing peroxidase to act on hydrogen peroxide and a chromogenic substrate to produce a color-forming dye is not limited. For example, peroxidase may be added to the reaction mixture in which hydrogen peroxide is produced (or assumed to be produced) by the reaction of step (2).

When application to an autoanalyzer is taken into consideration, the reagent components required for step (3) may be all prepared simultaneously with the reagent addition step in which all the reagent components required for step (2) are prepared, or in a previous reagent addition step. For example, for application to an autoanalyzer that can set two steps of reagent addition, the reagent components required for step (3) may be supplied so that they are all prepared in the first step that is started by the first reagent addition; however, they may be supplied so that they are all prepared in the second step. When the reagent components required for step (3) are supplied so that they are all prepared in the second step, they may be separately supplied in the first step and the second step.

For example, for application to an autoanalyzer that can set only one step of reagent addition, the reagent components required for step (3) and the reagent components required for step (2) may be supplied in the same step.

For application to an autoanalyzer that can set three or more steps of reagent addition, the method for supplying reagents may be suitably determined in consideration of the above principle.

1-7. Step (4) :Measurement of coloring amount etc.

In step (4), the amount of coloring of the color-forming dye obtained in step (3) is measured.

The measurement of the amount of coloring is performed by measuring absorbance changes arising from the amount of dye developed by the action of peroxidase on hydrogen peroxide and a chromogenic substrate within a suitable time range after step (3) is started.

Although the measurement wavelength of absorbance is not limited, the wavelength suitable for measurement differs depending on the dye. For example, when 10-(carboxymethylaminocarbonyl)-3,7-bis(dimethylamino)phenothiazine sodium salt is used as a chromogenic substrate, the measurement wavelength is preferably selected from the range of 600 to 700 run. For example, absorbance at 660 nm may be measured.

When N-(carboxymethylaminocarbonyl)-4,4'-bis(dimethylamino)diphenylamine sodium salt is used as a chromogenic substrate, the measurement wavelength is preferably selected from the range of 650 to 750 nm. For example, absorbance at 727 nm may be measured.

When 10-N-carboxymethylcarbamoyl-3,7-bis(dimethylamino)-10H-phenothiazine is used as a chromogenic substrate, the measurement wavelength is preferably selected from the range of 600 to 700 nm. For example, absorbance at 660 nm may be measured.

When 10-N-methylcarbamoyl-3,7-bis(dimethylamino)-10H-phenothiazine is used as a chromogenic substrate, the measurement wavelength is preferably selected from the range of 600 to 700 nm. For example, absorbance at 660 nm may be measured.

When 4,4'-bis(dimethylamino)diphenylamine and bis[3-bis(4-chlorophenyl)methyl-4-dimethylaminophenyl]amine are used as chromogenic substrates, the measurement wavelength is preferably selected from the range of 700 to 800 nm. For example, absorbance at 750 nm may be measured.

When N,N,N',N',N'',N''-hexa-3-sulfopropyl-4,4'4''-triaminotriphenylmethane is used as a chromogenic substrate, the measurement wavelength is preferably selected from the range of 550 to 650 nm. For example, absorbance at 591 nm may be measured.

When 4,4'-benzylidenebis(N,N-dimethylaniline) is used as a chromogenic substrate, the measurement wavelength is preferably selected from the range of 550 to 650 nm. For example, absorbance at 617 nm may be measured.

When 4,4'-bis(dimethylamino)diphenylamine is used as a chromogenic substrate, the measurement wavelength is preferably selected from the range of 650 to 750 nm. For example, absorbance at 700 nm may be measured.

In the case of an autoanalyzer that can set two steps of reagent addition, the measurement can be performed by, for example, measuring absorbance changes in the time range in which the second step is executed. The above timing is not limited. For example, absorbance is measured immediately after the addition of the second reagent, and a certain period of time after the addition of the second reagent (e.g., any timing between 60 seconds to 600 seconds, preferably after 300 seconds), and the absorbance change can be determined from their difference. In addition, various known absorbance change measurement methods (e.g., one-point end method, two-point end method, and rate method) may be used. These methods can be suitably set according to the instruction manual of each autoanalyzer.

1-8. Step (5): Calibrator, calibration method, etc.

In step (5), the amount of coloring obtained in step (4) is applied to a calibration curve of a glycated hemoglobin ratio and the amount of coloring previously created using one or more glycated hemoglobin calibrators value-assigned by one or more glycated hemoglobin ratios to determine a glycated hemoglobin ratio. As a typical calibration curve, for example, the glycated hemoglobin ratio is plotted on the x-axis, and the amount of coloring is plotted on the y-axis.

Calibration is a method in which a calibration curve is previously created using a calibrator for which the relationship between a value of the target measurement item (e.g., the glycated hemoglobin ratio in the present invention) and a measured value (e.g., the amount of coloring in the present invention) is known, the measured value of a sample is applied to the calibration curve, and the value of the measurement item of the sample is obtained. The calibrator is also called a standard reagent, a standard solution, or a calibration reagent.

The base composition of the calibrator used in the present invention is not limited. For example, components derived from human blood, or artificially produced components may be used. For components derived from human blood, whole blood may be used, or some components, such as blood cells, may be isolated and used.

Moreover, the form of these calibrators is not limited within the range that does not impair the function of freeze drying, liquid state, freezing, etc.

The solvent of the calibrator used in the present invention is not limited, and distilled water and buffers can be used. Examples include Good's buffers, carbonate buffers, phosphate buffers, citrate buffers, acetate buffers, borate buffers, tartrate buffers, and the like.

For the calibrator used in the present invention, various antibacterial agents and preservatives can be used, if necessary. Examples of preservatives include various types of ProClin (registered trademark of Supelco), azides, chelating agents, antibiotics, antibacterial agents, and the like. Examples of chelating agents include ethylenediaminetetraacetic acid, salts thereof, and the like. Examples of antibiotics include gentamycin, kanamycin, chloramphenicol, and the like. Examples of antibacterial agents include piperacillin, imidazolidinyl urea, and the like.

For the calibrator used in the present invention, various sugars, salts, and proteins can be used, if necessary (for example, as stabilizing agents).

Examples of salts include, but are not limited to, sodium chloride, sodium acetate, sodium hydroxide, potassium chloride, potassium acetate, potassium hydroxide, aluminum chloride, aluminum acetate, aluminum hydroxide, calcium chloride, calcium acetate, calcium hydroxide, and the like.

Examples of sugars include, but are not limited to, sucrose, trehalose, cyclodextrin, glycerol, gluconate, amino acids, and the like.

Examples of proteins that can be added include, but are not limited to, inactive proteins, such as serum albumins, globulins, and fibrous proteins. A preferable protein is bovine serum albumin.

In the calibrator used in the present invention, ferrocyanides, such as potassium ferrocyanide, and nitrites, such as sodium nitrite, may be added, if necessary (for example, for the purpose of avoiding the influence of reducing substances derived from the sample, or promoting the transformation of hemoglobin into methemoglobin, and the protease reaction), or may not be added.

In the calibrator used in present invention, a surfactant can be used, if necessary. Surfactants that can be used in the present invention are not limited. Usable examples include nonionic surfactants and/or amphoteric ionic surfactants and/or cationic surfactants and/or anionic surfactants.

Examples of nonionic surfactants include, but are not limited to, polyoxyethylene alkyl ether, fatty acid sorbitan ester, alkyl polyglucoside, fatty acid diethanolamide, alkyl monoglyceryl ether, and the like.

Examples of amphoteric ionic surfactants include, but are not limited to, alkyldimethyl amine oxide, alkyl carboxy betaine, and the like.

Examples of cationic surfactants include, but are not limited to, benzalkonium chloride, distearyldimethylbenzylammonium chloride, coconut amine acetate, stearyl amine acetate, and the like.

Examples of anionic surfactants include, but are not limited to, dipotassium alkenylsuccinate, and the like.

Some of the additives that can be used in the calibrator used in the present invention can also function as an anionic surfactant.

The concentrations and conditions of the above components (enzymes, chromogenic substrates, surfactants, and other components present in reagents) can be suitably determined, in consideration of reactivity and/or stability, etc., depending on the type of reagent, enzyme, etc., used.

The above determination does not need to be an optimal solution, and it is sufficient that performance practically sufficient for measurement of glycated hemoglobin ratio is ensured depending on the purpose and situation.

The calibrator used in the present invention is value-assigned by a glycated hemoglobin ratio. Generally used calibrators are those value-assigned by glycated hemoglobin ratios by various measurement methods after pooling of human blood; however, artificially glycated proteins (e.g., hemoglobin) or amino acids, or decomposition products thereof can be added to the above pooled serum to adjust glycated hemoglobin ratios.

The calibrator used in the present invention can be value-assigned by the glycated hemoglobin ratio of a reaction mixture by various measurement methods, such as HPLC method, enzymatic method, and latex immunization method, using standard substances, such as IFCC and NGSP.

The hemoglobin concentration can be measured by various measurement methods, such as cyanmethemoglobin method, acid hematin method, alkaline hematin method, oxyhemoglobin method, SLS-hemoglobin method, Hb-red measurement method, and HbCO measurement method.

In the present specification, it is preferable to follow a cyanmethemoglobin method using a Drabkin reagent. Specifically, a Drabkin reagent is prepared by dissolving 200 mg of potassium ferricyanide, 50 mg of potassium cyanide, and 1.0 g of sodium hydrogen carbonate in distilled water and adjusting it to 1 L. After that, 4.5 ml of Drabkin test solution and 0.5 ml of sample are added to a test tube and mixed, and the mixture is then allowed to stand at room temperature for 20 minutes. Then, the absorbance of cyano methemoglobin generated in the reaction mixture is measured at 540 nm.

The number of levels of value-assignment of the calibrators used in the present invention is not limited, as long as it is two or more levels (i.e., two or more levels with different glycated hemoglobin ratios). Two levels are preferable; however, three or more multiple levels (preferably five levels or less) may be used. For example, when calibrators value-assigned by two levels are used, it is possible to perform three levels of calibration using, in addition to these calibrators, a sample that does not contain glycated hemoglobin, such as purified water or a buffer, as a substitute for a calibrator, in combination with the two levels of the former calibrators during calibration.

The reason that two levels are preferable is that a larger number of levels requires larger amounts of reagents used, and is complicated.

In the case of two levels, the glycated hemoglobin ratios of the calibrators used in the present invention are two types of low concentration and high concentration. It is desirable that the low concentration is 3% to 7%, and that the high concentration is 8% to 20%; and it is more desirable that the low concentration is 3% to 6%, and that the high concentration is 8% to 20%. It is even more desirable that the low concentration is 3% to 6%, and that the high concentration is 10% to 20%; it is still more desirable that the low concentration is 3% to 5.5%, and that the high concentration is 10% to 20%; it is further still more desirable that the low concentration is 3% to 5%, and that the high concentration is 10% to 20%; and it is particularly desirable that the low concentration is 3% to 5%, and that the high concentration is 10% to 18%.

The calibrators used in the present invention are glycated hemoglobin calibrators value-assigned by two or more levels of glycated hemoglobin ratios, and when the lowest total hemoglobin concentration of the reaction mixture at the time of completion of measurement for value-assignment is regarded as 1, the other (all the remaining) total hemoglobin concentrations are preferably about 1 to 6 times, more preferably about 1 to 5 times, even more preferably about 1 to 4 times, still more preferably about 1 to 3.5 times, and particularly preferably about 1 to 3 times, the lowest total hemoglobin concentration. The other total hemoglobin concentrations are more preferably about 1 to 2.5 times, even more preferably about 1 to 2 times, still more preferably about 1 to 1.5 times, and particularly preferably about 1 time (equivalent concentration), the lowest total hemoglobin concentration.

The height order of total hemoglobin concentrations may be irrelevant to the height order of glycated hemoglobin ratios. For example, in the case of calibrators with three levels of glycated hemoglobin ratios (low concentration, medium concentration, and high concentration), any of their total hemoglobin concentrations may be highest or lowest. From the viewpoint that a more excellent correlation is highly likely to be obtained, it is preferable that a low glycated hemoglobin ratio is regarded as a low total hemoglobin concentration, and that a high glycated hemoglobin ratio is regarded as a high total hemoglobin concentration.

The calibrator used in the present invention may be one that is produced so that it is diluted when used to satisfy the above total hemoglobin concentration ratio.

The absolute value of the total hemoglobin concentration of the calibrator used in the present invention is not limited, but is preferably set so that the concentration thereof in the reaction mixture at the time of completion of measurement is 0.1 $\mu$mol/L or more (preferably 1 $\mu$mol/L or more, and more preferably 3 $\mu$mol/L or more), and so that the concentration thereof in the reaction mixture at the time of completion of measurement is 50 $\mu$mol/L or less (preferably 10 $\mu$mol/L or less, and more preferably 8 $\mu$mol/L or less).

The calibrator used in the present invention may be one that is produced so that it is diluted when used to satisfy the absolute value of the above total hemoglobin concentration ratio.

The total hemoglobin concentration of the calibrator alone is not limited. For example, the total hemoglobin concentration can be set to about 10 $\mu$mol/L to 900 $\mu$mol/L, preferably about 20 $\mu$mol/L to 700 $\mu$mol/L, more preferably about 30 $\mu$mol/L to 500 $\mu$mol/L, even more preferably about 40 $\mu$mol/L to 300 $\mu$mol/L, and still more preferably about 50 $\mu$mol/L to 250 $\mu$mol/L. Further, for example, a calibrator at 50 $\mu$mol/L to 200 $\mu$mol/L may be used as it is, or a calibrator at 2 mmol/L to 5 mmol/L may be used after dilution with water or a pretreatment liquid.

The absolute value of the glycated hemoglobin concentration of the calibrator used in the present invention is not limited. It is preferably set so that the concentration thereof in the reaction mixture at the time of completion of measurement is 0.01 $\mu$mol/L or more (preferably 0.05 $\mu$mol/L or more, and more preferably 0.03 $\mu$mol/L or more), and so that the concentration thereof in the reaction mixture at the time of completion of measurement is 15 $\mu$mol/L or less (preferably 10 $\mu$mol/L or less, and more preferably 1 $\mu$mol/L or less) .

The calibrator used in the present invention may be one that is produced so that it is diluted when used to satisfy the absolute value of the above total hemoglobin concentration ratio.

The glycated hemoglobin concentration of the calibrator alone is not limited. For example, a calibrator at about 1 $\mu$mol/L to 40 $\mu$mol/L can be used; preferably, a calibrator at about 1 $\mu$mol/L to 30 $\mu$mol/L can be used. Further, for example, a calibrator at 1 $\mu$mol/L to 20 $\mu$mol/L may be used as it is, or a calibrator at 100 $\mu$mol/L to 500 $\mu$mol/L may be used after dilution with water or a pretreatment liquid.

1-9. Method for measuring glycated hemoglobin ratio, and other matters

In the present invention, the reaction is preferably performed in a buffer. Examples of the buffer include, but are not limited to, Good's buffers, carbonate buffers, phosphate buffers, citrate buffers, acetate buffers, borate buffers, tartrate buffers, and the like.

In the present invention, various antibacterial agents and preservatives can be used, if necessary. Examples of preservatives include various types of ProClin (registered trademark of Supelco), azides, chelating agents, antibiotics, antibacterial agents, and the like. Examples of chelating agents include ethylenediaminetetraacetic acid, salts thereof, and the like. Examples of antibiotics include gentamycin, kanamycin, chloramphenicol, and the like. Examples of antibacterial agents include piperacillin, imidazolidinyl urea, and the like.

In the present invention, various sugars, salts, and proteins can be used, if necessary (for example, as enzyme stabilizers).

Examples of salts include, but are not limited to, sodium chloride, sodium acetate, sodium hydroxide, potassium chloride, potassium acetate, potassium hydroxide, aluminum chloride, aluminum acetate, aluminum hydroxide, calcium chloride, calcium acetate, calcium hydroxide, and the like.

Examples of sugars include, but are not limited to, sucrose, trehalose, cyclodextrin, glycerol, gluconate, amino acids, and the like.

Examples of proteins that can be added include, but are not limited to, inactive proteins, such as serum albumins, globulins, and fibrous proteins. A preferable protein is bovine serum albumin. Preferable inactive proteins are those that do not contain protease impurities that lead to enzymolysis.

In the present invention, ferrocyanides, such as potassium ferrocyanide, and nitrites, such as sodium nitrite, may be added, if necessary (for example, for the purpose of avoiding the influence of reducing substances derived from the sample, or promoting the transformation of hemoglobin into methemoglobin, and the protease reaction), or may not be added.

In the present invention, a surfactant can be used, if necessary. Surfactants that can be used in the present invention are not limited. Usable examples include nonionic surfactants and/or amphoteric ionic surfactants and/or cationic surfactants and/or anionic surfactants.

Examples of nonionic surfactants include, but are not limited to, polyoxyethylene alkyl ether, fatty acid sorbitan ester, alkyl polyglucoside, fatty acid diethanolamide, alkyl monoglyceryl ether, and the like.

Examples of amphoteric ionic surfactants include, but are not limited to, alkyldimethyl amine oxide, alkyl carboxy betaine, and the like.

Examples of cationic surfactants include, but are not limited to, benzalkonium chloride, distearyldimethylbenzylammonium chloride, coconut amine acetate, stearyl amine acetate, and the like.

Examples of anionic surfactants include, but are not limited to, dipotassium alkenylsuccinate, and the like.

Some of the additives that can be used in the present invention can also function as an anionic surfactant.

The concentrations and conditions of the above components (enzymes, chromogenic substrates, surfactants, and other components present in reagents) can be suitably determined, in consideration of reactivity and/or stability, etc., depending on the type of reagent, enzyme, etc., used.

In that case, it is preferable to take into consideration that there is a possibility that the measurement of glycated hemoglobin, the stability of reagents, etc., are affected by mutual interference of the components present in the reagents. For example, for application to an autoanalyzer that can set two steps of reagent addition, when protease and glycated amino acid oxidase coexist in the second step, the conditions can be suitably determined so that the characteristics of both enzymes can be utilized as much as possible.

The above determination does not need to be an optimal solution, and it is sufficient that performance practically sufficient for measurement of glycated hemoglobin ratio is ensured depending on the purpose and situation.


2. Kit for measuring glycated hemoglobin ratio

One embodiment of the present invention is a kit for measuring a glycated hemoglobin ratio, the kit comprising the following reagents (a) to (e):

(a) a reagent containing a compound having a sulfo group;
(b) a reagent containing glycated amino acid oxidase;
(c) a reagent containing peroxidase;
(d) a reagent containing a chromogenic substrate that produces a color-forming dye upon contact with hydrogen peroxide in the presence of peroxidase; and
(e) glycated hemoglobin calibrators value-assigned by two or more levels of glycated hemoglobin ratios, wherein the glycated hemoglobin calibrators value-assigned by two or more levels of glycated hemoglobin ratios are such that when the lowest total hemoglobin concentration at the time of completion of value-assignment is regarded as 1, the other total hemoglobin concentrations at the time of completion of value-assignment are 1 to 6 times the lowest total hemoglobin concentration.

The kit of the present invention may further comprise the following reagent (f):
(f) a reagent containing protease.

The structure of the kit for measuring a glycated hemoglobin ratio of the present invention is not limited. For example, the kit of the present invention may comprise instruction manuals etc., that describe, in recording media (e.g., paper), glycated amino acid oxidase, buffers, protease, peroxidase, chromogenic substrates, chelating reagents that capture ions blocking enzyme reaction, ascorbate oxidase that eliminates ascorbic acid, which is an interfering substance of qualitative analysis of hydrogen peroxide, surfactants, stabilizing agents, excipients, antibacterial agents, preservatives, and the measurement method of the present invention.

The kit for measuring a glycated hemoglobin ratio of the present invention can be used for the method for measuring a glycated hemoglobin ratio of the present invention. Therefore, the kit of the present invention may be constituted from articles that are used to carry out the measurement method of the present invention. About the description of the above articles, the description in the item of the measurement method of the present invention can be incorporated by reference.

The kit of the present invention may be constituted from several parts, in accordance with the form of an autoanalyzer. For example, in the case of a kit applicable to an autoanalyzer that can set two steps of reagent addition, the kit may be constituted from two parts, i.e., a first reagent and a second reagent.

When the storage stability of the kit is taken into consideration, the kit may be constituted from more than two parts, and may be configured so that two reagents, i.e., a first reagent and a second reagent, can be prepared immediately before use.

For application to an autoanalyzer that can set two steps of reagent addition, when an elimination system is constructed, it is preferable that the glycated hemoglobin ratio measurement kit of the present invention is constituted from two parts, that the first reagent contains glycated amino acid oxidase, and that the second reagent contains protease. In a reagent of such a form, the first reagent may further contain one or more of catalase, peroxidase, and dye.

In addition to the above structure, the kit of the present invention may further contain a sample pretreatment agent. The form of the pretreatment agent is not limited. Examples of the purpose of the pretreatment include, but are not limited to, dilution, hemolysis, and the like. A compound having a sulfo group may be added to a pretreatment agent, and the step of bringing the compound having a sulfo group into contact with the sample containing glycated hemoglobin (step (1)) may be performed during pretreatment.

About the kit described above, Table 1 shows combination examples of a pretreatment agent, first reagent, and second reagent for a compound having a sulfo group, glycated amino acid oxidase, peroxidase, a chromogenic substrate that produces a color-forming dye upon contact with hydrogen peroxide in the presence of peroxidase, and protease.

In the table, A represents a compound having a sulfo group, B represents glycated amino acid oxidase, C represents peroxidase, D represents a chromogenic substrate that produces a color-forming dye upon contact with hydrogen peroxide in the presence of peroxidase, and F represents protease.

Table 1

|  | Pretreatment agent | First reagent (R1) | Second reagent (R2) |
|---|---|---|---|
| Example 1 |  | A, C, F | B, D |
| Example 2 | A | C, F | B, D |
| Example 3 |  | A, B, D | C, F |
| Example 4 | A | B, D | C, F |
| Example 5 |  | A | B, C, D, F |
| Example 6 | A | B, C, D, F | None |
| Example 7 |  | A, B | C, D |
| Example 8 | A | B | C, D |
| Example 9 | A | B, C | D |
| Example 10 | A | B, C, D | None |
| Example 11 | F | A, D | B, C |
| Example 12 | F | A | B, C, D |

(continued)

|  | Pretreatment agent | First reagent (R1) | Second reagent (R2) |
|---|---|---|---|
| Example 13 | F | A, B, C, D | None |
| Example 14 | F | A, C | B, D |
| Example 15 | F | A, B, D | C |

A: compound having a sulfo group
B: glycated amino acid oxidase
C: peroxidase
D: chromogenic substrate that produces a color-forming dye upon contact with hydrogen peroxide in the presence of peroxidase
F: protease

The present invention is described in more detail below with reference to Examples; however, the present invention is not limited to the following Examples.

Examples

Example 1

The HbAlc ratio was determined by the following method,
1. Reagent
First reagent (R1)

| MES buffer (pH: 6.5): | 100 mM |
|---|---|
| $CaCl_2$: | 0.71 g/L |
| Protease: | 100 KU/L |
| Peroxidase: | 3.7 KU/L |
| Sodium dodecylbenzenesulfonate: | 3 g/L |

Second reagent (R2)

| MES buffer (pH: 6.5) : | 100 mM |
|---|---|
| Glycated amino acid oxidase: | 5 KU/L |
| DA-67: | 16 mg/L |

2. Sample
Blood cells: 10 samples
They were diluted 30 times with purified water before measurement.
3. Measurement parameter

Measurement model: Hitachi 7180
Analysis method/measurement point: 2 point end (10) 16-34

When this parameter is used, the time required for measurement is a total of about 600 seconds (first reaction: about 300 seconds, and second reaction: about 300 seconds).

Measurement wavelength (nm) (sub/main): 800/660
Liquid measure ratio: Sample/R1/R2 = 10 $\mu$L/120 $\mu$L/40 $\mu$L (S:R1:R2 = 1:12:4)
Reaction temperature: 37°C
ABS LIMIT 32000 increase
Calibration: calculated at 0.0-10000 absorbance [0111]

The above measurement results were compared with the following calibration curves A to D (x-axis: HbAlc ratio, y-axis: coloring amount) to determine the HbA1c ratio. The HbA1c ratio used herein is a value determined as a NGSP value.

The calibration curve A was created using a calibrator 1 (hemoglobin (hereinafter referred as Hb) : 66.0 $\mu$mol/L; HbA1c: 2.93 $\mu$mol/L; HbA1c ratio: 6.2%) and a calibrator 2 (Hb: 66.0 $\mu$mol/L; HbA1c: 7.34 $\mu$mol/L; HbA1c ratio: 12.3%).

The calibration curve B was created using a calibrator 3 (Hb: 78.8 $\mu$mol/L; HbA1c: 3.49 $\mu$mol/L; HbA1c ratio: 6.2%) and a calibrator 4 (Hb: 78.8 $\mu$mol/L; HbA1c: 8.75 $\mu$mol/L; HbA1c ratio: 12.3%).

The calibration curve C was created using a calibrator 5 (Hb: 56.9 $\mu$mol/L; HbA1c: 2.52 $\mu$mol/L; HbA1c ratio: 6.2%) and a calibrator 6 (Hb: 56.9 $\mu$mol/L; HbA1c: 6.32 $\mu$mol/L; HbA1c ratio: 12.3%).

The calibration curve D was created using a calibrator 7 (Hb: 50.0 $\mu$mol/L; HbA1c: 2.21 $\mu$mol/L; HbA1c ratio: 6.2%) and a calibrator 8 (Hb: 97.6 $\mu$mol/L; HbA1c: 10.83 $\mu$mol/L; HbA1c ratio: 12.3%).

HPLC method (control)

Measurements were carried out using Arkray HA8170 according to a general method.

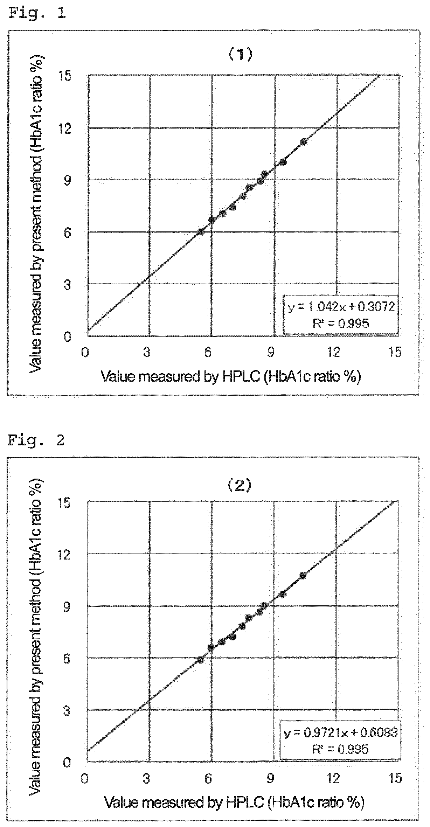

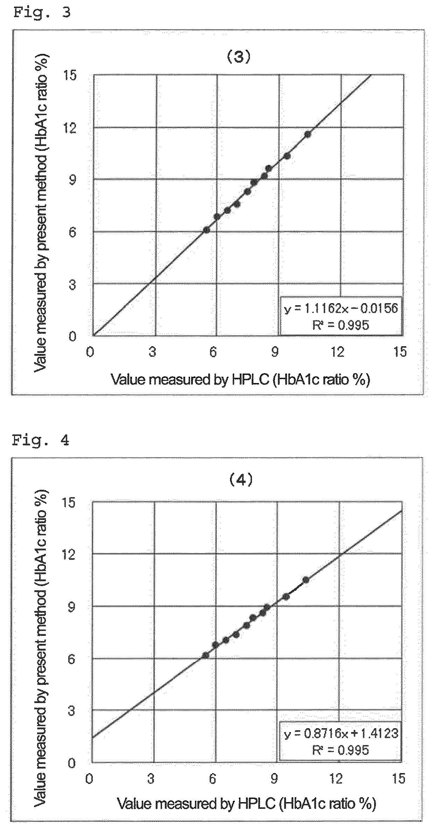

Figs. 1 to 4 show correlation data between the method of the present invention and the HPLC method. The correlation equations with the HPLC method when using the above calibration curves A to D were the following equations (1) to (4):

$$(1) \quad Y = 1.042X + 0.3072, \quad R^2 = 0.995$$

$$(2) \quad Y = 0.9721X + 0.6083, \quad R^2 = 0.995$$

$$(3) \quad Y = 1.1162X - 0.0156, \quad R^2 = 0.995$$

$$(4) \quad Y = 0.8716X + 1.4123, \quad R^2 = 0.995$$

Thus, the correlation between the method of the present invention and the HPLC method was very excellent.

Example 2 and Comparative Example 1

The HbA1c ratio was determined by the following method.

1. Reagent

First reagent (R1)

| | |
|---|---|
| HEPES buffer (pH: 7.3): | 100 mM |
| CaCl$_2$ : | 0.71 g/L |
| Protease: | 100 KU/L |
| Peroxidase: | 3.7 KU/L |
| Additives shown in Table 2: | 3 g/L |

Second reagent (R2)

| | |
|---|---|
| HEPES buffer (pH: 7.3): | 100 mM |
| Glycated amino acid oxidase: | 5 KU/L |
| DA-67: | 16 mg/L |

2. Sample

Blood cells: 10 samples

They were diluted 30 times with purified water before measurement.

3. Measurement parameter

The method described in Example 1 was used.

The above measurement results were compared with the calibration curve created using the calibrators 1 and 2 described in Example 1, and the calibration curves created using the reagents A to E shown in Table 2 to determine the HbA1c ratio.

As a Comparative Example, the calibration curve created using the calibrators 1 and 2 described in Example 1 was compared with the calibration curve created using the reagents F and G to determine the HbAlc ratio.

In these cases, the HbAlc ratio is a value determined as a NGSP value.

Table 2

|  |  | Additive | Figure |
|---|---|---|---|
| Example | Reagent A | 3-aminobenzenesulfonic acid | (5) |
|  | Reagent B | Sodium alkyl diphenyl ether sulfonate | (6) |
|  | Reagent C | Sodium alkylnaphthalenesulfonate | (7) |
|  | Reagent D | Sulfosuccinic acid di-2-ethylhexyl sodium salt | (8) |
|  | Reagent E | Sodium dialkylsulfosuccinate | (9) |
| Comparative Example | Reagent F | No additives | (10) |
|  | Reagent G | Potassium ferricyanide | (11) |

HPLC method (control)

Measurements were carried out using Arkray HA8170 according to a general method.

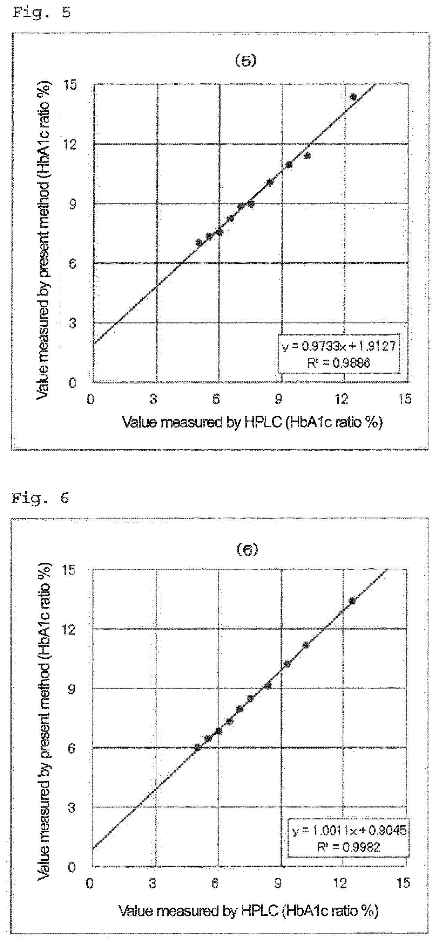

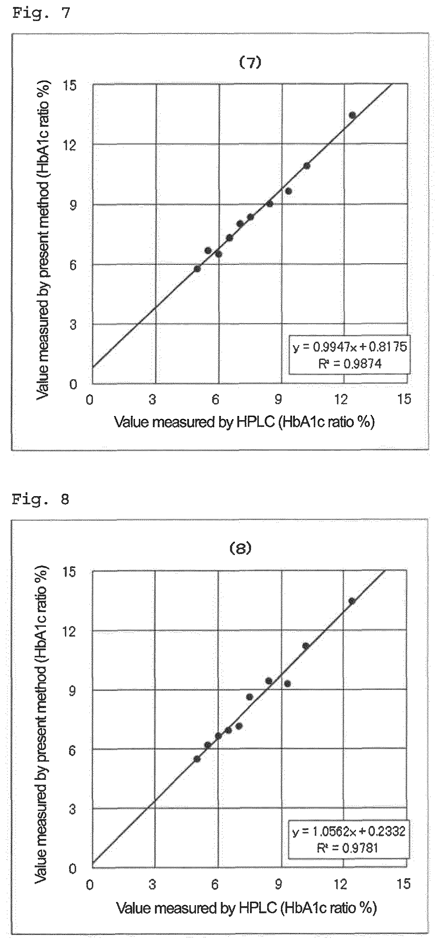

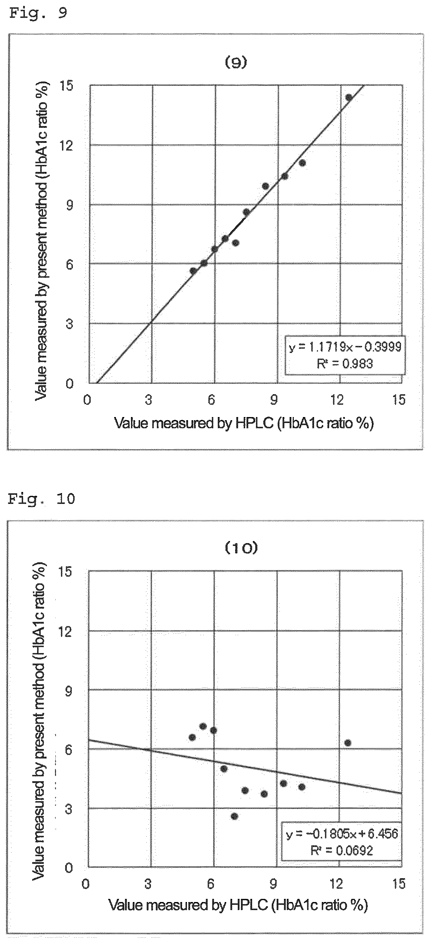

Figs. 5 to 11 show correlation data between the method of the present invention and the HPLC method. The correlation equations with the HPLC method when using the above reagents A to G were the following equations (5) to (11):

$$(5)\ Y = 0.9733X + 1.9127,\ R^2 = 0.9886$$

$$(6)\ Y = 1.0011X + 0.9045,\ R^2 = 0.9982$$

$$(7)\ Y = 0.9947X + 0.8175,\ R^2 = 0.9874$$

$$(8)\ Y = 1.0562X + 0.2332,\ R^2 = 0.9781$$

$$(9)\ Y = 1.1719X - 0.3999,\ R^2 = 0.9830$$

$$(10)\ Y = -0.1805X + 6.456,\ R^2 = 0.0692$$

$$(11)\ Y = 0.593X + 1.7699,\ R^2 = 0.3080$$

Thus, the correlation between the method of the present invention and the HPLC method was very excellent ((5) to (9)). In contrast, no correlation was recognized in the Comparative Examples ((10) and (11)).

Example 3

The HbAlc ratio was determined by the following method.

1. Reagent

First reagent (R1)
MES buffer (pH: 6.5): 100 mM
CaCl$_2$: 0.71 g/L
Surfactant: 1 g/L
Protease: 100 KU/L
DA-67: 16 mg/L

Sodium dodecylbenzenesulfonate: 3 g/L
Second reagent (R2)
MES buffer (pH: 6.5): 100 mM
Glycated amino acid oxidase: 5 KU/L
Peroxidase: 3.7 KU/L

2. Sample
Blood cells: 30 samples
They were diluted 30 times with purified water before measurement.
3. Measurement parameter

Measurement model: Hitachi 7180
Analysis method/measurement point: 2 point end (10) 16-34

When this parameter is used, the time required for measurement is a total of about 600 seconds (first reaction: about 300 seconds, and second reaction: about 300 seconds).

Measurement wavelength (nm) (sub/main): 800/660
Liquid volume ratio: Sample/RI/R2 = 10 μL/120 μL/40 μL (S:R1:R2 = 1:12:4)
Reaction temperature: 37°C
ABS LIMIT 32000 increase
Calibration: calculated at 0.0-10000 absorbance

The above measurement results were compared with the following calibration curves E to H (x-axis: HbAlc ratio, y-axis: coloring amount) to determine the HbAlc ratio. The HbAlc ratio used herein is a value determined as a NGSP value.
The calibration curve E was created using a calibrator 9 (hemoglobin (hereinafter referred to as Hb): 210.5 μmol/L; HbAlc: 7.0 μmol/L; HbAlc ratio: 5.2%) and a calibrator 10 (Hb: 210.5 μmol/L; HbAlc: 19.3 μmol/L; HbAlc ratio: 10.5%).
The calibration curve F was created using a calibrator 11 (Hb: 421.1 μmol/L; HbAlc: 14.0 μmol/L; HbAlc ratio: 5.2%) and a calibrator 12 (Hb: 210.5 μmol/L; HbAlc: 19.3 μmol/L; HbAlc ratio: 10.5%).
The calibration curve G was created using a calibrator 13 (Hb: 634.9 μmol/L; HbAlc: 21.2 μmol/L; HbAlc ratio: 5.2%) and a calibrator 14 (Hb: 210.5 μmol/L; HbAlc: 19.3 μmol/L; HbAlc ratio: 10.5%).
The calibration curve H was created using a calibrator 15 (Hb: 833.3 μmol/L; HbA1c: 27.8 μmol/L; HbA1c ratio: 5.2%) and a calibrator 16 (Hb: 210.5 μmol/L; HbAlc: 19.3 μmol/L; HbAlc ratio: 10.5%).

HPLC method (control)

Measurements were carried out using Arkray HA8170 according to a general method.

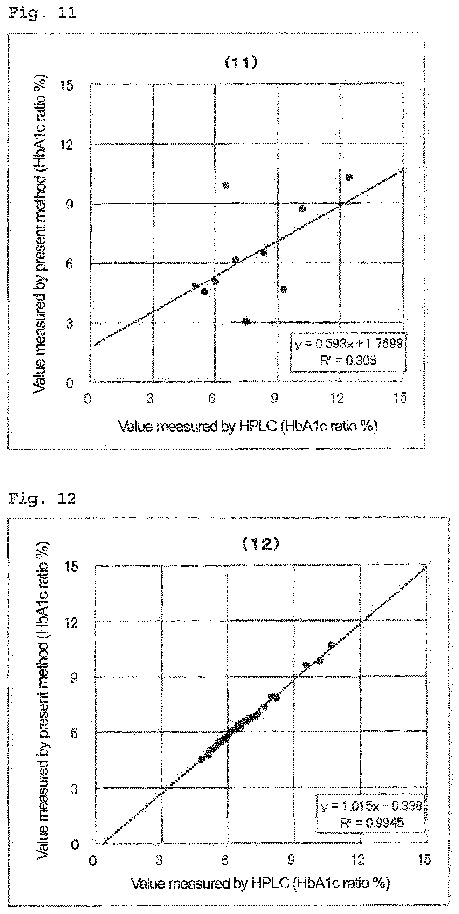

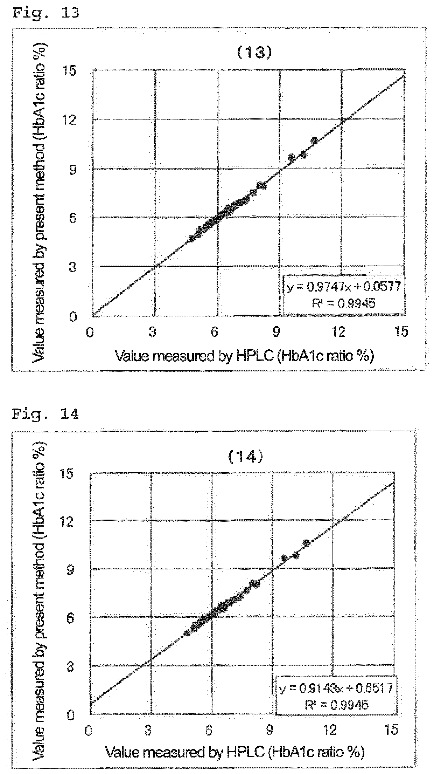

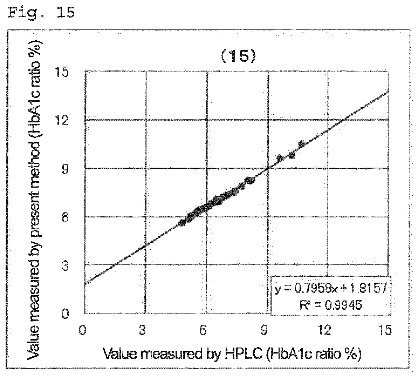

Figs. 12 to 15 show correlation data between the method of the present invention and the HPLC method. The correlation equations with the HPLC method when using the above calibration curves E to H were the following equations (12) to (15):

$$(12) \quad Y = 1.015X - 0.338, \quad R^2 = 0.9945$$

$$(13) \quad Y = 0.9747X + 0.0577, \quad R^2 = 0.9945$$

$$(14) \quad Y = 0.9143X + 0.6517, \quad R^2 = 0.9945$$

$$(15) \quad Y = 0.7958X + 1.8157, \quad R^2 = 0.9945$$

Thus, the correlation between the method of the present invention and the HPLC method was very excellent. In particular, it was clarified that particularly high correlation was observed when the total hemoglobin concentration ratio of the glycated hemoglobin calibrator was about 1:1 to 3.

Industrial Applicability

The present invention contributes to, for example, diagnosis and medical fields, including measurement of a glycated hemoglobin ratio, which is useful as a diabetes marker etc.

**Claims**

1. A method for measuring a glycated hemoglobin ratio, the method comprising the following steps (1) to (5):

(1) bringing a sample containing glycated hemoglobin into contact with a compound having a sulfo group;
(2) allowing glycated amino acid oxidase to act on the sample containing glycated hemoglobin to produce hydrogen peroxide;
(3) bringing the hydrogen peroxide produced in step (2) into contact with a chromogenic substrate in the presence of peroxidase to produce a color-forming dye;
(4) measuring the amount of coloring of the color-forming dye obtained in step (3);
(5-1) creating a calibration curve of a glycated hemoglobin ratio and the amount of coloring using glycated hemoglobin calibrators value-assigned by two or more levels of glycated hemoglobin ratios, wherein the glycated hemoglobin calibrators value-assigned by two or more levels of glycated hemoglobin ratios are such that when the lowest total hemoglobin concentration at the time of completion of value-assignment is regarded as 1, the other total hemoglobin concentrations at the time of completion of value-assignment are 1 to 6 times the lowest total hemoglobin concentration; and
(5-2) applying the amount of coloring obtained in step (4) to the calibration curve created in step (5-1) to determine a glycated hemoglobin ratio.

2. The measurement method according to claim 1, wherein the glycated hemoglobin calibrators value-assigned by two or more levels of glycated hemoglobin ratios used in step (5-1) are such that when the lowest total hemoglobin concentration is regarded as 1, the other total hemoglobin concentrations are 1 to 3 times the lowest total hemoglobin concentration.

3. The measurement method according to claim 1 or 2, further comprising the following step (6):
(6) decomposing the glycated hemoglobin into a glycated hemoglobin decomposition product by the action of protease.

4. The measurement method according to any one of claims 1 to 3, further comprising the following step (7):
(7) hemolyzing the sample containing glycated hemoglobin.

5. The measurement method according to any one of claims 1 to 4, wherein the compound having a sulfo group used in step (1) is at least one compound selected from the group consisting of benzenesulfonic acid, alkyl sulfonic acid, naphthalenesulfonic acid, sulfosuccinic acid, benzothiazoline sulfonic acid, anthraquinone sulfonic acid, camphor sulfonic acid, benzoxadiazole sulfonic acid, derivatives thereof, and salts thereof.

6. The measurement method according to any one of claims 1 to 5, wherein the compound having a sulfo group used in step (1) is at least one compound selected from the group consisting of dodecylbenzenesulfonic acid, 3-aminobenzenesulfonic acid, alkyldiphenyl ether sulfonic acid, 4-aminoazobenzene-4'-sulfonic acid, 4-amino-4'-nitrostilbene-2,2'-disulfonic acid, 4,4'-diazostilbene-2,2'-disulfonic acid, sulfanilic acid, 5-sulfoisophthalic acid, 5-amino-2-chlorotoluene-4-sulfonic acid, alkylnaphthalenesulfonic acid, 2-naphthalenesulfonic acid, 8-amino-1-naphthalenesulfonic acid, dodecylnaphthalenesulfonic acid, di-2-ethylhexyl sulfosuccinate, dialkylsulfosuccinic acid, and salts thereof.

7. The measurement method according to any one of claims 1 to 6, wherein the chromogenic substrate used in step (3) is a leuco chromogen.

8. The measurement method according to any one of claims 1 to 7, wherein the chromogenic substrate used in step (3) is a phenothiazine-based leuco chromogen.

9. The measurement method according to any one of claims 1 to 8, wherein the chromogenic substrate used in step (3) is at least one phenothiazine-based leuco chromogen selected from the group consisting of 10-(carboxymethylaminocarbonyl)-3,7-bis(dimethylamino)phenothiazine sodium salt, 10-N-carboxymethylcarbamoyl-3,7-bis(dimethylamino)-10H-phenothiazine, and 10-N-methylcarbamoyl-3,7-bis(dimethylamino)-10H-phenothiazine.

10. The measurement method according to any one of claims 1 to 9, wherein the chromogenic substrate used in step (3) is 10-(carboxymethylaminocarbonyl)-3,7-bis(dimethylamino)phenothiazine sodium salt.

11. The measurement method according to any one of claims 1 to 10, which is performed in one channel of an autoanalyzer.

12. A kit for measuring a glycated hemoglobin ratio, the kit comprising the following reagents (a) to (e):

(a) a reagent containing a compound having a sulfo group;
(b) a reagent containing glycated amino acid oxidase;
(c) a reagent containing peroxidase;
(d) a reagent containing a chromogenic substrate that produces a color-forming dye upon contact with hydrogen peroxide in the presence of peroxidase; and
(e) glycated hemoglobin calibrators value-assigned by two or more levels of glycated hemoglobin ratios, wherein the glycated hemoglobin calibrators value-assigned by two or more levels of glycated hemoglobin ratios are such that when the lowest total hemoglobin concentration at the time of completion of value-assignment is regarded as 1, the other total hemoglobin concentrations at the time of completion of value-assignment are 1 to 6 times the lowest total hemoglobin concentration.

13. The measurement kit according to claim 12, wherein the glycated hemoglobin calibrators value-assigned by two or more levels of glycated hemoglobin ratios (e) are such that when the lowest total hemoglobin concentration is regarded as 1, the other total hemoglobin concentrations are 1 to 3 times the lowest total hemoglobin concentration.

14. The measurement kit according to claim 12 or 13, further comprising the following reagent (f):
(f) a reagent containing protease.

**Patentansprüche**

1. Verfahren zum Messen eines Verhältnisses von glykiertem Hämoglobin, wobei das Verfahren die folgenden Schritte (1) bis (5) umfasst:

(1) Inkontaktbringen einer Probe, die glykiertes Hämoglobin enthält, mit einer Verbindung, die eine Sulfogruppe aufweist;
(2) Ermöglichen einer glykierten Aminosäureoxidase, auf die Probe, die glykiertes Hämoglobin enthält, zu wirken, um Wasserstoffperoxid zu erzeugen;
(3) Inkontaktbringen des in Schritt (2) erzeugten Wasserstoffperoxids mit einem chromogenen Substrat in Anwesenheit von Peroxidase, um einen farbbildenden Farbstoff zu erzeugen;
(4) Messen der Menge der Färbung des in Schritt (3) erhaltenen farbbildenden Farbstoffs;
(5-1) Erstellen einer Kalibrierungskurve eines Verhältnisses von glykiertem Hämoglobin und der Menge der Färbung unter Verwendung von Kalibratoren von glykiertem Hämoglobin, denen durch zwei oder mehrere Grade von Verhältnissen von glykiertem Hämoglobin Werte zugeordnet wurden, wobei die Kalibratoren von glykiertem Hämoglobin, denen durch zwei oder mehrere Grade von Verhältnissen von glykiertem Hämoglobin Werte zugeordnet wurden, derart sind, dass wenn die geringste Gesamtkonzentration von Hämoglobin zum Zeitpunkt der Beendigung der Wertezuordnung als 1 angesehen wird, die anderen Gesamtkonzentrationen von Hämoglobin zum Zeitpunkt der Beendigung der Wertezuordnung das 1- bis 6-Fache der niedrigsten Gesamtkonzentration von Hämoglobin sind; und
(5-2) Anwenden der in Schritt (4) erhaltenen Menge an Färbung auf die in Schritt (5-1) erstellte Kalibrierungskurve, um ein Verhältnis von glykiertem Hämoglobin zu bestimmen.

2. Messverfahren nach Anspruch 1, wobei die Kalibratoren von glykiertem Hämoglobin, denen durch zwei oder mehrere in Schritt (5-1) verwendete Grade von Verhältnissen von glykiertem Hämoglobin Werte zugeordnet wurden, derart sind, dass wenn die geringste Gesamtkonzentration von Hämoglobin als 1 angesehen wird, die anderen Gesamtkonzentrationen von Hämoglobin das 1- bis 3-Fache der niedrigsten Gesamtkonzentration von Hämoglobin sind.

3.  Messverfahren nach Anspruch 1 oder 2, des Weiteren umfassend den folgenden Schritt (6):
    (6) Abbauen des glykierten Hämoglobins in ein Abbauprodukt von glykiertem Hämoglobin durch das Wirken einer Protease.

4.  Messverfahren nach einem der Ansprüche 1 bis 3, des Weiteren umfassend den folgenden Schritt (7):
    (7) Hämolyse der Probe, die glykiertes Hämoglobin enthält.

5.  Messverfahren nach einem der Ansprüche 1 bis 4, wobei die in Schritt (1) verwendete Verbindung, die eine Sulfogruppe aufweist, mindestens eine Verbindung ist, die ausgewählt ist aus der Gruppe bestehend aus Benzolsulfonsäure, Alkylsulfonsäure, Naphthalinsulfonsäure, Sulfobernsteinsäure, Benzothiazolinsulfonsäure, Anthrachinonsulfonsäure, Camphersulfonsäure, Benzoxadiazolsulfonsäure, Derivaten davon und Salzen davon.

6.  Messverfahren nach einem der Ansprüche 1 bis 5, wobei die in Schritt (1) verwendete Verbindung, die eine Sulfogruppe aufweist, mindestens eine Verbindung ist, die ausgewählt ist aus der Gruppe bestehend aus Dodecylbenzolsulfonsäure, 3-Aminobenzolsulfonsäure, Alkyldiphenylethersulfonsäure, 4-Aminoazobenzol-4'-sulfonsäure, 4-Amino-4'-nitrostilben-2,2'-disulfonsäure, 4,4'-Diazostilben-2,2'-disulfonsäure, Sulfanilsäure, 5-Sulfoisophthalsäure, 5-Amino-2-chlortoluol-4-sulfonsäure, Alkylnaphthalinsulfonsäure, 2-Naphthalinsulfonsäure, 8-Amino-1-naphthalinsulfonsäure, Dodecylnaphthalinsulfonsäure, Di-2-ethylhexylsulfosuccinat, Dialkylsulfobernsteinsäure und Salzen davon.

7.  Messverfahren nach einem der Ansprüche 1 bis 6, wobei das in Schritt (3) verwendete chromogene Substrat ein Leukochromogen ist.

8.  Messverfahren nach einem der Ansprüche 1 bis 7, wobei das in Schritt (3) verwendete chromogene Substrat ein Leukochromogen auf Phenothiazinbasis ist.

9.  Messverfahren nach einem der Ansprüche 1 bis 8, wobei das in Schritt (3) verwendete chromogene Substrat mindestens ein Leukochromogen auf Phenothiazinbasis ist, das ausgewählt ist aus der Gruppe bestehend aus 10-(Carboxymethylaminocarbonyl)-3,7-bis(dimethylamino)phenothiazin-Natriumsalz, 10-N-Carboxymethylcarbamoyl-3,7-bis(dimethylamino)-10H-phenothiazin und 10-N-Methylcarbamoyl-3,7-bis(dimethylamino)-10H-phenothiazin.

10. Messverfahren nach einem der Ansprüche 1 bis 9, wobei das in Schritt (3) verwendete chromogene Substrat 10-(Carboxymethylaminocarbonyl)-3,7-bis(dimethylamino)phenothiazin-Natriumsalz ist.

11. Messverfahren nach einem der Ansprüche 1 bis 10, das in einem Kanal eines Autoanalyzers durchgeführt wird.

12. Kit zum Messen eines Verhältnisses von glykiertem Hämoglobin, wobei der Kit die folgenden Reagenzien (a) bis (e) umfasst:

    (a) ein Reagenz, das eine Verbindung enthält, die eine Sulfogruppe aufweist;
    (b) ein Reagenz, das glykierte Aminosäureoxidase enthält;
    (c) ein Reagenz, das Peroxidase enthält;
    (d) ein Reagenz, das ein chromogenes Substrat enthält, das bei Kontakt mit Wasserstoffperoxid in Gegenwart von Peroxidase einen farbbildenden Farbstoff erzeugt; und
    (e) Kalibratoren von glykiertem Hämoglobin, denen durch zwei oder mehrere Grade von Verhältnissen von glykiertem Hämoglobin Werte zugeordnet wurden, wobei die Kalibratoren von glykiertem Hämoglobin, denen durch zwei oder mehrere Grade von Verhältnissen von glykiertem Hämoglobin Werte zugeordnet wurden, derart sind, dass wenn die geringste Gesamtkonzentration von Hämoglobin zum Zeitpunkt der Beendigung der Wertezuordnung als 1 angesehen wird, die anderen Gesamtkonzentrationen von Hämoglobin zum Zeitpunkt der Beendigung der Wertezuordnung das 1- bis 6-Fache der niedrigsten Gesamtkonzentration von Hämoglobin sind.

13. Messkit nach Anspruch 12, wobei die Kalibratoren von glykiertem Hämoglobin, denen durch zwei oder mehrere Grade von Verhältnissen von glykiertem Hämoglobin Werte zugeordnet wurden (e), derart sind, dass wenn die geringste Gesamtkonzentration von Hämoglobin als 1 angesehen wird, die anderen Gesamtkonzentrationen von Hämoglobin das 1- bis 3-Fache der niedrigsten Gesamtkonzentration von Hämoglobin sind.

# EP 3 550 030 B1

**14.** Messkit nach Anspruch 12 oder 13, des Weiteren umfassend das folgende Reagens (f): (f) ein Reagens, das Protease enthält.

**Revendications**

**1.** Procédé pour mesurer le taux d'hémoglobine glyquée, le procédé comprenant les étapes (1) à (5) suivantes :

(1) un échantillon contenant de l'hémoglobine glyquée est mis en contact avec un composé ayant un groupe sulfo ;
(2) de l'acide aminé glyqué oxydase est laissée à agir sur l'échantillon contenant de l'hémoglobine glyquée pour produire du peroxyde d'hydrogène ;
(3) le peroxyde d'hydrogène produit dans l'étape (2) est mis en contact avec un substrat chromogénique en présence de peroxydase pour produire un colorant chromogène ;
(4) la quantité de coloration du colorant chromogène obtenu dans l'étape (3) est mesurée ;
(5-1) une courbe d'étalonnage du taux d'hémoglobine glyquée et de la quantité de coloration est créée par utilisation d'étalons d'hémoglobine glyquée dont les valeurs ont été attribuées par deux ou plus de deux niveaux de taux d'hémoglobine glyquée, lesquels étalons d'hémoglobine glyquée dont les valeurs ont été attribuées par deux ou plus de deux niveaux de taux d'hémoglobine glyquée sont tels que, lorsque la concentration d'hémoglobine totale minimale au moment de l'achèvement de l'attribution des valeurs est considérée être de 1, les autres concentrations d'hémoglobine totale au moment de l'achèvement de l'attribution de valeurs sont de 1 à 6 fois la concentration d'hémoglobine totale minimale ; et
(5-2) la quantité de coloration obtenue dans l'étape (4) est appliquée à la courbe d'étalonnage créée dans l'étape (5-1) pour qu'un taux d'hémoglobine glyquée soit déterminé.

**2.** Procédé de mesure selon la revendication 1, dans lequel les étalons d'hémoglobine glyquée dont les valeurs ont été attribuées par deux ou plus de deux niveaux de taux d'hémoglobine glyquée utilisés dans l'étape (5-1) sont tels que, lorsque la concentration d'hémoglobine totale minimale est considérée être de 1, les autres concentrations d'hémoglobine totale sont de 1 à 3 fois la concentration d'hémoglobine totale minimale.

**3.** Procédé de mesure selon la revendication 1 ou 2, comprenant en outre l'étape (6) suivante :
(6) l'hémoglobine glyquée est décomposée en un produit de décomposition d'hémoglobine glyquée par l'action d'une protéase.

**4.** Procédé de mesure selon l'une quelconque des revendications 1 à 3, comprenant en outre l'étape (7) suivante :
(7) l'échantillon contenant de l'hémoglobine glyquée est hémolysé.

**5.** Procédé de mesure selon l'une quelconque des revendications 1 à 4, dans lequel le composé ayant un groupe sulfo utilisé dans l'étape (1) est au moins un composé choisi dans le groupe constitué par les suivants : acide benzènesulfonique, acide alkylsulfonique, acide naphtalènesulfonique, acide sulfosuccinique, acide benzothiazolinesulfonique, acide anthraquinonesulfonique, acide camphosulfonique, acide benzozadiazolesulfonique, leurs dérivés, et leurs sels.

**6.** Procédé de mesure selon l'une quelconque des revendications 1 à 5, dans lequel le composé ayant un groupe sulfo utilisé dans l'étape (1) est au moins un composé choisi dans le groupe constitué par les suivants : acide dodécylbenzènesulfonique, acide 3-aminobenzènesulfonique, acide alkyldiphényléthersulfonique, acide 4-aminoazobenzène-4'-sulfonique, acide 4-amino-4'-nitrostilbène-2,2'-disulfonique, acide 4,4'-diazostilbène-2,2'-disulfonique, acide sulfanilique, acide 5-sulfoisophtalique, acide 5-amino-2-chlorotoluène-4-sulfonique, acide alkylnaphtalènesulfonique, acide 2-naphtalènesulfonique, acide 8-amino-1-naphtalènesulfonique, acide dodécylnaphtalènesulfonique, sulfosuccinate de di-2-éthylhexyle, acide dialkylsulfosuccinique, et leurs sels.

**7.** Procédé de mesure selon l'une quelconque des revendications 1 à 6, dans lequel le substrat chromogénique utilisé dans l'étape (3) est un chromogène leuco.

**8.** Procédé de mesure selon l'une quelconque des revendications 1 à 7, dans lequel le substrat chromogénique utilisé dans l'étape (3) est un chromogène leuco à base de phénothiazine.

**9.** Procédé de mesure selon l'une quelconque des revendications 1 à 8, dans lequel le substrat chromogénique utilisé

24

dans l'étape (3) est au moins un chromogène leuco à base de phénothiazine choisi dans le groupe constitué par les suivants : sel sodique de 10-(carboxyméthylaminocarbonyl)-3,7-bis(diméthylamino)phénothiazine, 10-N-carboxyméthylcarbamoyl-3,7-bis(diméthylamino)-10H-phénothiazine, et 10-N-méthylcarbamoyl-3,7-bis(diméthylamino)-10H-phénothiazine.

10. Procédé de mesure selon l'une quelconque des revendications 1 à 9, dans lequel le substrat chromogénique utilisé dans l'étape (3) est le sel sodique de 10-(carboxyméthylaminocarbonyl)-3,7-bis(diméthylamino)phénothiazine.

11. Procédé de mesure selon l'une quelconque des revendications 1 à 10, qui est réalisé dans un seul canal d'un auto-analyseur.

12. Trousse pour mesurer le taux d'hémoglobine glyquée, la trousse comprenant les réactifs (a) à (e) suivants :

(a) un réactif contenant un composé ayant un groupe sulfo ;
(b) un réactif contenant une acide aminé glyquée oxydase ;
(c) un réactif contenant une peroxydase ;
(d) un réactif contenant un substrat chromogénique qui produit un colorant chromogène suite au contact avec du peroxyde d'hydrogène en présence de peroxydase ; et
(e) étalons d'hémoglobine glyquée dont les valeurs ont été attribuées par deux ou plus de deux niveaux de taux d'hémoglobine glyquée, lesquels étalons d'hémoglobine glyquée dont les valeurs ont été attribuées par deux ou plus de deux niveaux de taux d'hémoglobine glyquée sont tels que, lorsque la concentration d'hémoglobine totale minimale au moment de l'achèvement de l'attribution des valeurs est considérée être de 1, les autres concentrations d'hémoglobine totale au moment de l'achèvement de l'attribution de valeurs sont de 1 à 6 fois la concentration d'hémoglobine totale minimale.

13. Trousse de mesure selon la revendication 12, dans laquelle les étalons d'hémoglobine glyquée dont les valeurs ont été attribuées par deux ou plus de deux niveaux de taux d'hémoglobine glyquée (e) sont tels que, lorsque la concentration d'hémoglobine totale minimale est considérée être de 1, les autres concentrations d'hémoglobine totale sont de 1 à 3 fois la concentration d'hémoglobine totale minimale.

14. Trousse de mesure selon la revendication 12 ou 13, comprenant en outre le réactif (f) suivant :
(f) un réactif contenant une protéase.

Fig. 1

(1)

y = 1.042x + 0.3072
R² = 0.995

Fig. 2

(2)

y = 0.9721x + 0.6083
R² = 0.995

Fig. 3

(3)

y = 1.1162x − 0.0156
R² = 0.995

Fig. 4

(4)

y = 0.8716x + 1.4123
R² = 0.995

Fig. 5

Fig. 6

Fig. 7

(7)

y = 0.9947x + 0.8175
R² = 0.9874

Value measured by present method (HbA1c ratio %)

Value measured by HPLC (HbA1c ratio %)

Fig. 8

(8)

y = 1.0562x + 0.2332
R² = 0.9781

Value measured by present method (HbA1c ratio %)

Value measured by HPLC (HbA1c ratio %)

Fig. 9

Fig. 10

Fig. 11

Fig. 12

Fig. 13

(13)

Fig. 14

(14)

Fig. 15

(15)

y = 0.7958x + 1.8157
R² = 0.9945

Value measured by present method (HbA1c ratio %)

Value measured by HPLC (HbA1c ratio %)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008108385 A
- JP 2007147630 A
- EP 1002874 A
- JP 4427137 B

- WO 08108385 A
- WO 2015005257 A
- WO 2015005258 A
- WO 2015060429 A